# EUROPEAN PATENT SPECIFICATION

(11) **EP 2 646 572 B1**
(45) Date of publication and mention of the grant of the patent: **15.02.2017**
(21) Application number: 11790963.0
(22) Date of filing: 30.11.2011
(51) Int. Cl.: C12Q 1/68

(54) **DIAGNOSTIC AND TREATMENT OF CHRONIC HEART FAILURE**
DIAGNOSE UND BEHANDLUNG VON CHRONISCHEM HERZVERSAGEN
DIAGNOSTIC ET TRAITEMENT D'UNE INSUFFISANCE CARDIAQUE CHRONIQUE

(30) Priority: 01.12.2010 EP 10306338
(43) Date of publication of application: 09.10.2013
(73) Proprietor: INSERM (Institut National de la Santé et de la Recherche Médicale), 75013 Paris (FR)
(72) Inventor: SMIH, Fatima, F-31432 Toulouse Cedex 4 (FR); ROUET, Philippe, F-31432 Toulouse Cedex 4 (FR)
(74) Representative: Cabinet Plasseraud
(86) International application number: PCT/EP2011/071383
(87) International publication number: WO 2012/072681

(56) References cited:
- WO-A2-2004/050894
- WO-A2-2006/052857
- LAMANT M ET AL: "ApoO, a novel apolipoprotein, is an original glycoprotein up-regulated by diabetes in human heart", JOURNAL OF BIOLOGICAL CHEMISTRY 20061124 AMERICAN SOCIETY FOR BIOCHEMISTRY AND MOLECULAR BIOLOGY INC. US, vol. 281, no. 47, 24 November 2006 (2006-11-24), pages 36289-36302, XP002627267, DOI: DOI:10.1074/JBC.M510861200
- LAMANT M. ET AL: "108 Localization and regulation of a new Apolipoprotein in human heart: A possible protective mechanism to lipotoxicity?", EUROPEAN JOURNAL OF HEART FAILURE SUPPLEMENTS, ELSEVIER, vol. 4, no. 1, 1 June 2005 (2005-06-01), page 25, XP004990890, ISSN: 1567-4215
- STEENMAN M ET AL: "Distinct molecular portraits of human failing hearts identified by dedicated cDNA microarrays", EUROPEAN JOURNAL OF HEART FAILURE, ELSEVIER, AMSTERDAM, NL, vol. 7, no. 2, 2 March 2005 (2005-03-02), pages 157-165, XP004739458, ISSN: 1388-9842, DOI: DOI:10.1016/J.EJHEART.2004.05.008
- DOUGLAS S A ET AL: "Congestive heart failure and expression of myocardial urotensin II", LANCET THE, LANCET LIMITED. LONDON, GB, vol. 359, no. 9322, 8 June 2002 (2002-06-08), pages 1990-1997, XP004795091, ISSN: 0140-6736, DOI: DOI:10.1016/S0140-6736(02)08831-1

## Description

### FIELD OF THE INVENTION:

The invention describes Apolipoprotein O (ApoO) as a biomarker for chronic heart failure. Moreover, the invention relates to a compound which is an inhibitor of the Apo O gene expression for use in the treatment of chronic heart failure.

### BACKGROUND OF THE INVENTION:

Heart failure is a common, costly, disabling, and potentially deadly condition. In developed countries, around 2% of adults suffer from heart failure, but in those over the age of 65, this increases to 6-10%. Heart failure is associated with significantly reduced physical and mental health, resulting in a markedly decreased quality of life. Although some people survive many years, progressive disease is associated with an overall annual mortality rate of 10%. So, there is a permanent need in the art for new molecules for the treatment and new diagnostic of heart failure and especially for chronic heart failure (see for example Dickstein et al., 2008).

Lamant et al. have discovered a new Apolipoprotein in human heart, named Apo O. (Lamant et al. European Journal of Heart Failure Supplements, vol 4, no1, page 25, June 2005; Lamant et al. JBC, vol 281, no47, pages 36289-36302, November 2006).

WO2006052857 describes beta-adrenergic receptor kinase (βARK1) as a biomarker for heart failure.

WO2004050894 describes almost a hundred candidate target genes for heart failure. Steenman et al. (European Journal of Heart Failure, vol 7, no2, pages 157-165, March 2005), mentions that distinct molecular portaits of human failing hearts identified by dedicated cDNA microarrays.

Douglas et al. (The Lancet, vol 359, no9322, pages 1990-1997, June 2002) describes that urotensin II might have role in chronic heart failure.

### SUMMARY OF THE INVENTION:

The inventors show that ApoO expression, a recently identified protein, is strongly positively correlated with lipid metabolism, apoptosis, ATP synthesis and ROS synthesis in cardiac myoblastes or cardiomyocytes.

Surprisingly, the inventors discovered that the ApoO gene or protein may be used as a biomarker for the diagnostic of chronic heart failure and that an inhibitor of ApoO activity or gene expression may be used in the treatment of chronic heart failure.

The specification describes a method for diagnosis of chronic heart failure in a patient comprising a step consisting of detecting Apolipoprotein O (Apo O) expression in a sample obtained from said patient. Moreover, the invention relates to a compound which is an inhibitor of the Apo O gene expression for use in the treatment of chronic heart failure.

### DETAILED DESCRIPTION OF THE INVENTION:

### Definitions:

Throughout the specification, several terms are employed and are defined in the following paragraphs.

As used herein, the term "Apolipoprotein O" or "ApoO" has its general meaning in the art and denotes a member of the Apolipoprotein proteins family that bind to lipids to form lipoproteins, which transport the lipids through the lymphatic and circulatory systems. ApoO is a 198-amino acid protein that contains a 23-amino acid long signal peptide. The apolipoproteinO gene is expressed in a set of human tissues. An exemplary sequence for human ApoO gene is deposited in the database under accession number NM_024122. An exemplary sequence for human ApoO protein is deposited in the UniProtKB/Swiss-Prot database under accession numbers Q9BUR5 and C9J574 because of alternative splicing.

As used herein, the term "chronic heart failure" denotes inability of the heart to supply sufficient blood flow to meet the body's needs and this pathology is well-described in medicine practice. This term encompasses chronic heart failure, acute heart failure, and diabetic cardiomyopathy.

### Diagnostic method

The specification describes a method for diagnosis of chronic heart failure in a patient comprising a step consisting of detecting Apolipoprotein O (Apo O) expression in a sample obtained from said patient.

The chronic heart failure may be a systolic chronic heart failure or a diastolic chronic heart failure.

Typically, the sample according to the invention may be a blood, plasma, serum, lymph or urine sample. In a preferred embodiment, said sample is blood or urine.

The term "detecting" as used above includes qualitative and/or quantitative detection (measuring levels) with or without reference to a control. Typically ApoO expression may be measured for example by RT-PCR or immunohistochemistry performed on the sample.

The specification describes a method for diagnosis of chronic heart failure in a patient comprising a step a) consisting of measuring Apolipoprotein O (ApoO) expression in a sample obtained from said patient. Preferably, the method of the invention further comprises a step of comparing the ApoO expression level obtained in step a) to a threshold level.

The "control" may be a healthy subject, i.e. a subject who does not suffer from any chronic heart failure. The control may also be a subject suffering from chronic heart failure. Preferably, said control is a healthy subject.

For example detecting ApoO expression in sample may be performed by measuring the expression level of ApoO gene.

Typically, the detection comprises contacting the sample with selective reagents such as probes, primers or ligands, and thereby detecting the presence, or measuring the amount, of polypeptides or nucleic acids of interest originally present in the sample. Contacting may be performed in any suitable device, such as a plate, microtiter dish, test tube, well, glass, column... In specific embodiments, the contacting is performed on a substrate coated with the reagent, such as a nucleic acid array or a specific ligand array. The substrate may be a solid or semi-solid substrate such as any suitable support comprising glass, plastic, nylon, paper, metal, polymers and the like. The substrate may be of various forms and sizes, such as a slide, a membrane, a bead, a column, a gel, etc. The contacting may be made under any condition suitable for a detectable complex, such as a nucleic acid hybrid or an antibody-antigen complex, to be formed between the reagent and the nucleic acids or polypeptides of the sample.

In a particular embodiment, the expression level of ApoO gene may be determined by determining the quantity of mRNA of ApoO gene. Such method may be suitable to measure the expression level of ApO gene in the sample.

Methods for measuring the quantity of mRNA are well known in the art. For example the nucleic acid contained in the samples (e.g., cell or tissue prepared from the patient) is first extracted according to standard methods, for example using lytic enzymes or chemical solutions or extracted by nucleic-acid-binding resins following the manufacturer's instructions. The extracted mRNA may be then detected by hybridization (e. g., Northern blot analysis).

Alternatively, the extracted mRNA may be subjected to coupled reverse transcription and amplification, such as reverse transcription and amplification by polymerase chain reaction (RT-PCR), using specific oligonucleotide primers that enable amplification of a region in the ApoO gene. Preferably quantitative or semi-quantitative RT-PCR is used. Real-time quantitative or semi-quantitative RT-PCR is particularly advantageous. Extracted mRNA may be reverse-transcribed and amplified, after which amplified sequences may be detected by hybridization with a suitable probe or by direct sequencing, or any other appropriate method known in the art.

Other methods of amplification include ligase chain reaction (LCR), transcription-mediated amplification (TMA), strand displacement amplification (SDA) and nucleic acid sequence based amplification (NASBA).

Nucleic acids having at least 10 nucleotides and exhibiting sequence complementarity or homology to the mRNA of interest herein find utility as hybridization probes or amplification primers. It is understood that such nucleic acids need not be identical, but are typically at least about 80% identical to the homologous region of comparable size, more preferably at least 85% identical and even more preferably at least 90%, preferably at least 95% identical. In certain embodiments, it will be advantageous to use nucleic acids in combination with appropriate means, such as a detectable label, for detecting hybridization. A wide variety of appropriate indicators are known in the art including, fluorescent, radioactive, enzymatic or other ligands (e. g. avidin/biotin).

Probes typically comprise single-stranded nucleic acids of between 10 to 1000 nucleotides in length, for instance of between 10 and 800, more preferably of between 15 and 700, typically of between 20 and 500. Primers typically are shorter single-stranded nucleic acids, of between 10 to 25 nucleotides in length, designed to perfectly or almost perfectly match a nucleic acid of interest, to be amplified. The probes and primers are "specific" to the nucleic acids they hybridize to, i.e. they preferably hybridize under high stringency hybridization conditions (corresponding to the highest melting temperature Tm, e.g., 50 % formamide, 5x or 6x SCC. SCC is a 0.15 M NaCl, 0.015 M Na-citrate).

The method described above can comprise the steps of providing total RNAs obtained from the sample of the patient, and subjecting the RNAs to amplification and hybridization to specific probes, more particularly by means of a quantitative or semi-quantitative RT-PCR.

Total RNAs can be easily extracted from the sample. For instance, the sample may be treated prior to its use, e.g. in order to render nucleic acids available. Techniques of cell or protein lysis, concentration or dilution of nucleic acids, are known by the skilled person.

Alternatively, the expression level of Apo O gene may be measured by DNA microarray analysis. Such DNA microarray or nucleic acid microarray consists of different nucleic acid probes that are chemically attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes comprise nucleic acids such as cDNAs or oligonucleotides that may be about 10 to about 60 base pairs. To measure the expression level of ApoO gene, a sample from a test subject, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art (see e.g. the review by Hoheisel, Nature Reviews, Genetics, 2006, 7:200-210).

Detection of ApoO expression in the sample may also be performed by measuring the level of ApoO protein. In the present application, the "level of ApoO protein" means the quantity or concentration of said ApoO protein.

Such methods comprise contacting a sample with a binding partner capable of selectively interacting with ApoO protein present in the sample. The binding partner is generally an antibody that may be polyclonal or monoclonal, preferably monoclonal.

The presence of the protein can be detected using standard electrophoretic and immunodiagnostic techniques, including immunoassays such as competition, direct reaction, or sandwich type assays. Such assays include, but are not limited to, Western blots; agglutination tests; enzyme-labeled and mediated immunoassays, such as ELISAs; biotin/avidin type assays; radioimmunoassays; immunoelectrophoresis; immunoprecipitation, etc. The reactions generally include revealing labels such as fluorescent, chemiluminescent, radioactive, enzymatic labels or dye molecules, or other methods for detecting the formation of a complex between the antigen and the antibody or antibodies reacted therewith. More preferably, determination of the concentrations of cholesterol and lactate are performed with a fluorescence-activated cell sorter (FACS). Said fluorescence-activated cell sorter is a machine that can rapidly separate the cells in a suspension on the basis of size and the color of their fluorescence.

The aforementioned assays generally involve separation of unbound protein in a liquid phase from a solid phase support to which antigen-antibody complexes are bound. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, and the like.

More particularly, an ELISA method can be used, wherein the wells of a microtiter plate are coated with a set of antibodies against the proteins to be tested. A sample containing or suspected of containing the marker protein is then added to the coated wells. After a period of incubation sufficient to allow the formation of antibody-antigen complexes, the plate(s) can be washed to remove unbound moieties and a detectably labeled secondary binding molecule is added. The secondary binding molecule is allowed to react with any captured sample marker protein, the plate is washed and the presence of the secondary binding molecule is detected using methods well known in the art.

One preferred method utilizes immunohistochemistry, a staining method based on immunoenzymatic reactions using monoclonal or polyclonal antibodies to detect cells or specific proteins such as tissue antigens. Typically, immunohistochemistry protocols involve at least some of the following steps:
1) antigen retrieval (eg., by pressure cooking, protease treatment, microwaving, heating in appropriate buffers, etc.);
2) application of primary antibody (i.e. anti-ApoO protein antibody) and washing;
3) application of a labeled secondary antibody that binds to primary antibody (often a second antibody conjugate that enables the detection in step 5) and wash;
4) an amplification step may be included;
5) application of a detection reagent (e.g. chromagen, fluorescently tagged molecule or any molecule having an appropriate dynamic range to achieve the level of or sensitivity required for the assay);
6) counterstaining may be used and
7) detection using a detection system that makes the presence of the proteins visible (to either the human eye or an automated analysis system), for qualitative or quantitative analyses.

Various immunoenzymatic staining methods are known in the art for detecting a protein of interest. For example, immunoenzymatic interactions can be visualized using different enzymes such as peroxidase, alkaline phosphatase, or different chromogens such as DAB, AEC, or Fast Red; or fluorescent labels such as FITC, Cy3, Cy5, Cy7, Alexafluors, etc. Counterstains may include H&E, DAPI, Hoechst, so long as such stains are compatable with other detection reagents and the visualization strategy used. As known in the art, amplification reagents may be used to intensify staining signal. For example, tyramide reagents may be used. The staining methods of the present invention may be accomplished using any suitable method or system as would be apparent to one of skill in the art, including automated, semi-automated or manual systems.

The method described above may comprise a further step consisting of comparing ApoO expression with a control reference.

The specification describes a method for diagnosis chronic heart failure in a patient comprising determining the expression level of Apolipoprotein O (ApoO) in a sample obtained from said patient and comparing said expression level to a threshold value. As used herein, "expression level of ApoO" refers to an amount or a concentration of a transcription product, for instance mRNA coding for ApoO, or of a translation product, for instance the protein ApoO. Typically, a level of mRNA expression can be expressed in units such as transcripts per cell or nanograms per microgram of tissue. A level of a polypeptide can be expressed as nanograms per microgram of tissue or nanograms per milliliter of a culture medium, for example. Alternatively, relative units can be employed to describe an expression level.

In a preferred embodiment, when the measure of ApoO gene expression is performed by rt qPCR, the expression level of ApoO gene in a patient suffering of chronic heart failure is increased by at least 35%, preferably by at least 40%, preferably by at least 50%; preferably by at least 60 %, preferably by at least 70%, preferably by at least 80%, more preferably by at least 90%, even more at least 100% compared to a control reference. In other words, preferably, when ApoO gene expression is measured by rt qPCR, the quantity of mRNA encoding ApoO gene in a patient suffering of chronic heart failure is increased by at least 35%, preferably by at least 40%, preferably by at least 50%; preferably by at least 60 %, preferably by at least 70%, preferably by at least 80%, more preferably by at least 90%, even more at least 100% compared to a control reference.

Expression levels of ApoO have been measured by RT-Real-time PCR method in white blood cells and expressed as of ApoO mRNA / 18S (Arbitrary Unit; AU). The inventors have established a threshold value for expression levels of ApoO to easily diagnose chronic heart failure. Preferably, this threshold value is comprised between about 100 and about 250, preferably between about 130 and about 220, preferably between about 150 and about 190, preferably between about 160 and about 180, most preferably said threshold value is about 173.

Typically, a "threshold value", "threshold level" or "cut-off value" can be determined experimentally, empirically, or theoretically. A threshold value can also be arbitrarily selected based upon the existing experimental and/or clinical conditions, as would be recognized by a person of ordinary skilled in the art. Preferably, the person skilled in the art may compare the expression levels of ApoO obtained according to the method of the invention with a defined threshold value.

Preferably, said threshold value is the mean expression level of ApoO of a population of healthy individuals. As used herein, the term "healthy individual" denotes a human which is known to be healthy, i.e. which does not suffer from chronic heart failure, has never been subjected to such chronic heart failure, and does not need any medical care.

Typically, the skilled person in the art may determine the expression level of ApoO in a biological sample, preferably blood, of 100 individuals known to be healthy. The mean value of the obtained expression levels is then determined, according to well known statistical analysis, so as to obtain the mean expression level of ApoO. Said value is then considered as being normal and thus constitute a threshold value. By comparing the expression levels of ApoO to this threshold value, the physician is then able to diagnose chronic heart failure. Indeed, by comparing the expression level of ApoO obtained in a biological sample, preferably blood or urine, of a given subject to a threshold value, one can easily determine whether said subject suffers from chronic heart failure or not.

Accordingly, the physician would be able to adapt and optimize appropriate medical care of a subject in a critical and life-threatening condition suffering from chronic heart failure. The determination of said prognosis is highly appropriate for follow-up care and clinical decision making.

Therefore, the specification describes a method for diagnosis of chronic heart failure in a patient comprising the following steps:
a) determining the level of expression of Apolipoprotein O (ApoO) in a sample obtained from said patient;
b) determining the mean expression level of ApoO in a biological sample of a population of healthy individuals, preferably 100 healthy individuals; and
c) a step of comparing the expression level of ApoO obtained of a) to the mean expression level of ApoO obtained in b).

The present specification describes to kits for the diagnosis of chronic heart failure, comprising means for detecting ApoO expression.

The kits may comprise an anti-ApoO protein antibody; and another molecule coupled with a signalling system which binds to said ApoO protein antibody.

Typically, the antibodies or combination of antibodies are in the form of solutions ready for use. In one embodiment, the kit comprises containers with the solutions ready for use. Any other forms are possibleand the man skilled in the art can routinely adapt the form to the use in immunohistochemistry.

Also described is the Apolipoprotein O (ApoO) gene or protein as a biomarker for the diagnosis of chronic heart failure.

Also described is an in vitro method for monitoring a patient's response to chronic heart failure treatment which comprises a step of measuring the expression level of ApoO gene, or a step of measuring the level of ApoO protein, in a sample from a patient.

Thus, the present specification provides for the use of ApoO gene or protein as a biomarker for the monitoring of anti chronic heart failure therapies.

The expression level of ApoO gene or the level of ApoO protein may be determined to monitor a patient's response to chronic heart failure treatment.

### Inhibitors and uses thereof

The invention relates to a compound which is an inhibitor of the ApoO gene expression for the treatment of chronic heart failure.

As used herein, the term "ApoO activity" denotes the capacity to the ApoO to bind to lipids to transport their through the lymphatic and circulatory systems or to enhance lipid uptake and lipid metabolism within cells.

Inhibitors of ApoO activity may be a low molecular weight inhibitor, e. g. a small organic molecule (natural or not).

The term "small organic molecule" refers to a molecule (natural or not) of a size comparable to those organic molecules generally used in pharmaceuticals. The term excludes biological macromolecules (e. g., proteins, nucleic acids, etc.). Preferred small organic molecules range in size up to about 5000 Da, more preferably up to 2000 Da, and most preferably up to about 1000 Da.

Inhibitors of ApoO activity can be an antibody which binds to the ApoO protein and inhibits the ApoO activity.

Antibodies directed against the ApoO protein can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies against the ApoO protein can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see, e.g., U.S. Pat. No. 4,946,778) can be adapted to produce anti-ApoO single chain antibodies. ApoO inhibitors useful in practicing the present invention also include anti-ApoO antibody fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to the ApoO.

Humanized anti-ApoO antibodies and antibody fragments thereof may also be prepared according to known techniques. "Humanized antibodies" are forms of non-human (e.g., rodent) chimeric antibodies that contain minimal sequence derived from non-human immunoglobulin. For the most part, humanized antibodies are human immunoglobulins (recipient antibody) in which residues from a hypervariable region (CDRs) of the recipient are replaced by residues from a hypervariable region of a non-human species (donor antibody) such as mouse, rat, rabbit or nonhuman primate having the desired specificity, affinity and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. Methods for making humanized antibodies are described, for example, by Winter (U.S. Pat. No. 5,225,539) and Boss (Celltech, U.S. Pat. No. 4,816,397).

ApoO activity inhibitors may be selected from aptamers. Aptamers are a class of molecules that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. and Gold L., 1990. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A that are selected from combinatorial libraries by two hybrid methods (Colas et al., 1996).

In one embodiment, small inhibitory RNAs (siRNAs) can function as inhibitors of ApoO gene expression for use in the present invention. ApoO gene expression can be reduced by contacting a subject or cell with a small double stranded RNA (dsRNA), or a vector or construct causing the production of a small double stranded RNA, such that ApoO gene expression is specifically inhibited (i.e. RNA interference or RNAi). Methods for selecting an appropriate dsRNA or dsRNA-encoding vector are well known in the art for genes whose sequence is known (e.g. see Tuschl, T. et al. (1999); Elbashir, S. M. et al. (2001); Hannon, GJ. (2002); McManus, MT. et al. (2002); Brummelkamp, TR. et al. (2002); U.S. Pat. Nos. 6,573,099 and 6,506,559; and International Patent Publication Nos. WO 01/36646, WO 99/32619, and WO 01/68836).

Ribozymes can also function as inhibitors of ApoO gene expression for use in the present invention. Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of ApoO mRNA sequences are thereby useful within the scope of the present invention. Specific ribozyme cleavage sites within any potential RNA target are initially identified by scanning the target molecule for ribozyme cleavage sites, which typically include the following sequences, GUA, GUU, and GUC. Once identified, short RNA sequences of between about 15 and 20 ribonucleotides corresponding to the region of the target gene containing the cleavage site can be evaluated for predicted structural features, such as secondary structure, that can render the oligonucleotide sequence unsuitable. The suitability of candidate targets can also be evaluated by testing their accessibility to hybridization with complementary oligonucleotides, using, e.g., ribonuclease protection assays.

Both antisense oligonucleotides and ribozymes useful as inhibitors of ApoO gene expression can be prepared by known methods. These include techniques for chemical synthesis such as, e.g., by solid phase phosphoramadite chemical synthesis. Alternatively, anti-sense RNA molecules can be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters. Various modifications to the oligonucleotides of the invention can be introduced as a means of increasing intracellular stability and half-life. Possible modifications include but are not limited to the addition of flanking sequences of ribonucleotides or deoxyribonucleotides to the 5' and/or 3' ends of the molecule, or the use of phosphorothioate or 2'-O-methyl rather than phosphodiesterase linkages within the oligonucleotide backbone.

Antisense oligonucleotides siRNAs and ribozymes of the invention may be delivered in vivo alone or in association with a vector. In its broadest sense, a "vector" is any vehicle capable of facilitating the transfer of the antisense oligonucleotide siRNA or ribozyme nucleic acid to the cells and preferably cells expressing ApoO. Preferably, the vector transports the nucleic acid to cells with reduced degradation relative to the extent of degradation that would result in the absence of the vector. In general, the vectors useful in the invention include, but are not limited to, plasmids, phagemids, viruses, other vehicles derived from viral or bacterial sources that have been manipulated by the insertion or incorporation of the the antisense oligonucleotide siRNA or ribozyme nucleic acid sequences. Viral vectors are a preferred type of vector and include, but are not limited to nucleic acid sequences from the following viruses: retrovirus, such as moloney murine leukemia virus, harvey murine sarcoma virus, murine mammary tumor virus, and rouse sarcoma virus; adenovirus, adeno-associated virus; SV40-type viruses; polyoma viruses; Epstein-Barr viruses; papilloma viruses; herpes virus; vaccinia virus; polio virus; and RNA virus such as a retrovirus. One can readily employ other vectors not named but known to the art.

Preferred viral vectors are based on non-cytopathic eukaryotic viruses in which non-essential genes have been replaced with the gene of interest. Non-cytopathic viruses include retroviruses (e.g., lentivirus), the life cycle of which involves reverse transcription of genomic viral RNA into DNA with subsequent proviral integration into host cellular DNA. Retroviruses have been approved for human gene therapy trials. Most useful are those retroviruses that are replication-deficient (i.e., capable of directing synthesis of the desired proteins, but incapable of manufacturing an infectious particle). Such genetically altered retroviral expression vectors have general utility for the high-efficiency transduction of genes in vivo. Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, 1990 and in Murry, 1991).

Preferred viruses for certain applications are the adeno-viruses and adeno-associated viruses, which are double-stranded DNA viruses that have already been approved for human use in gene therapy. The adeno-associated virus can be engineered to be replication deficient and is capable of infecting a wide range of cell types and species. It further has advantages such as, heat and lipid solvent stability; high transduction frequencies in cells of diverse lineages, including hemopoietic cells; and lack of superinfection inhibition thus allowing multiple series of transductions. Reportedly, the adeno-associated virus can integrate into human cellular DNA in a site-specific manner, thereby minimizing the possibility of insertional mutagenesis and variability of inserted gene expression characteristic of retroviral infection. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

Other vectors include plasmid vectors. Plasmid vectors have been extensively described in the art and are well known to those of skill in the art. See e.g. Sambrook et al., 1989. In the last few years, plasmid vectors have been used as DNA vaccines for delivering antigen-encoding genes to cells in vivo. They are particularly advantageous for this because they do not have the same safety concerns as with many of the viral vectors. These plasmids, however, having a promoter compatible with the host cell, can express a peptide from a gene operatively encoded within the plasmid. Some commonly used plasmids include pBR322, pUC18, pUC19, pRC/CMV, SV40, and pBlueScript. Other plasmids are well known to those of ordinary skill in the art. Additionally, plasmids may be custom designed using restriction enzymes and ligation reactions to remove and add specific fragments of DNA. Plasmids may be delivered by a variety of parenteral, mucosal and topical routes. For example, the DNA plasmid can be injected by intramuscular, eye, intradermal, subcutaneous, or other routes. It may also be administered by intranasal sprays or drops, rectal suppository and orally. It may also be administered into the epidermis or a mucosal surface using a gene-gun. The plasmids may be given in an aqueous solution, dried onto gold particles or in association with another DNA delivery system including but not limited to liposomes, dendrimers, cochleate and microencapsulation.

In a preferred embodiment, the antisense oligonucleotide, siRNA, shRNA or ribozyme nucleic acid sequence is under the control of a heterologous regulatory region, e.g., a heterologous promoter.. The promoter can also be, e.g., a viral promoter, such as CMV promoter or any synthetic promoters.
In a preferred embodiment, the invention relates to a shRNA, for use in the treatment of chronic heart failure. Said shRNA acts as an inhibitor of Apo O activity or an inhibitor of the Apo O gene expression. Preferably, said shRNA are from MISSION shRNA set and are selected from sh4 (TRCN 72707) and Sh5 (TRCN 72705), marketed by Sigma Aldrich, Saint Quentin Fallavier.

Also described is a method for treating chronic heart failure comprising administering to a subject in need thereof a therapeutically effective amount of compound which is an inhibitor of Apo O protein or an inhibitor of the Apo O gene expression.

### Therapeutic composition

Another object of the invention relates to a therapeutic composition comprising a compound according to the invention for the treatment of a chronic heart failure.

In a preferred embodiment, the therapeutic composition according to the invention may be used for the treatment of a systolic chronic heart failure or a diastolic chronic heart failure.

Any therapeutic agent of the invention may be combined with pharmaceutically acceptable excipients, and optionally sustained-release matrices, such as biodegradable polymers, to form therapeutic compositions.

"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

The form of the pharmaceutical compositions, the route of administration, the dosage and the regimen naturally depend upon the condition to be treated, the severity of the illness, the age, weight, and sex of the patient, etc.

The pharmaceutical compositions of the invention can be formulated for a topical, oral, intranasal, parenteral, intraocular, intravenous, intramuscular or subcutaneous administration and the like.

Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment.

In addition, other pharmaceutically acceptable forms include, e.g. tablets or other solids for oral administration; time release capsules; and any other form currently can be used.

The invention will be further illustrated by the following figures and examples.

### FIGURES:

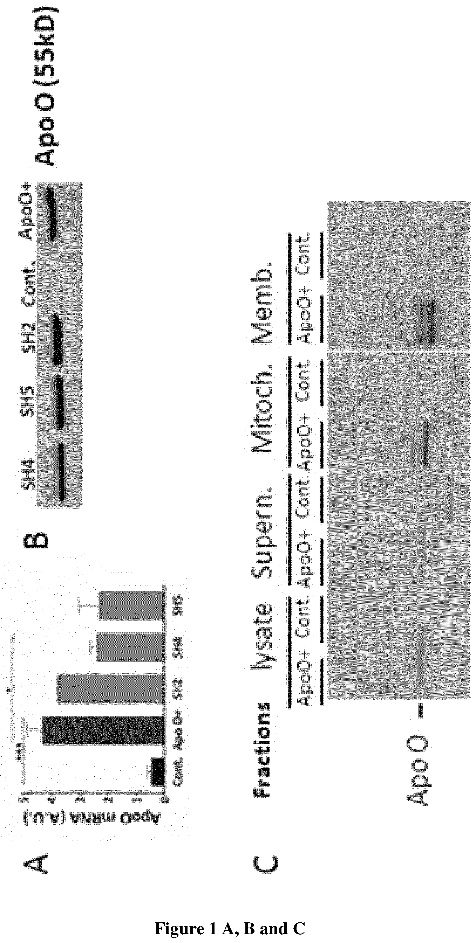

**Figure 1****: Human ApoO overexpression and intracellular localization in H9c2 rat cardiac myoblasts.**
   (A) Stable integration of ApoO expression vector in H9c2 cardiac myoblasts. (B) Knockdown of ApoO overexpression by SHRNA (SH2-5). Cont: control generated by stable integration of the expression vector without ApoO coding sequence. ApoO+: stable integration of pTT-ApoO expression vector; SH2-5: Stable integration of 3 ApoO sHRNA expression cassettes in the Apo O+ clone. ApoO mRNA levels were quantified by qPCRWestern blot analysis of ApoO expression in the different clones. (C) Western blot analysis of control and Apo O+ subcellular fractionation. Total cell lysate, supernatant, mitochondria and cytoplasmic membranes preparations are shown.
**Figure 2****: Analysis of Affymetrix microarrays human heart gene expression data extracted from GEO database.**
   (A) Distribution of ApoO expression within 90 human heart samples. Expression level is indicated in arbitrary units (A.U.). (B) Ingenuity pathway analysis of genes expression levels correlated to ApoO expression. A threshold is set to discard unsignificant correlations.
**Figure 3****: Mitochondrial and functional alterations induced by ApoO overexpression.**
   (A) Transmission electron microscopy of wild type and (B, C, D) ApoO transgenic mice hearts slices. Arrows are pointing alterated mitochondria. (E) Transthoracic echocardiographic analysis of wild type mice and ApoO transgenic (ApoO Tg). VTs: Telesystolic volume of the left ventricle. (F) ECG analysis of a set of randomly chosen wild type and ApoO transgenic mices. PR interval bar chart analysis of wild type and transgenic ApoO mice. ***p<0.01. (G, H, I, J) FACS analysis of the mitochondrial membrane potential in control (cont. and Apo O+ H9c2 cardiac myoblasts incubated without or with 100 µM palmitate. (K) Bar chart analysis of FACS data *p<0.05; **p<0.01 ; ***p<0.001.

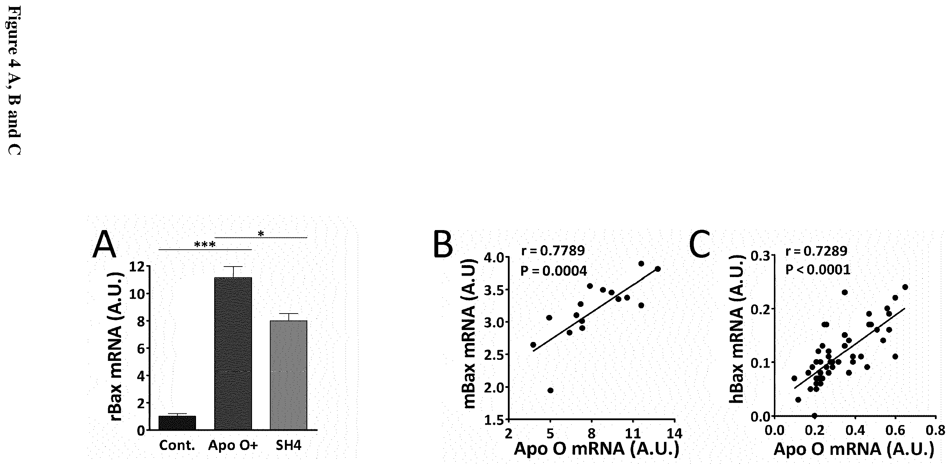

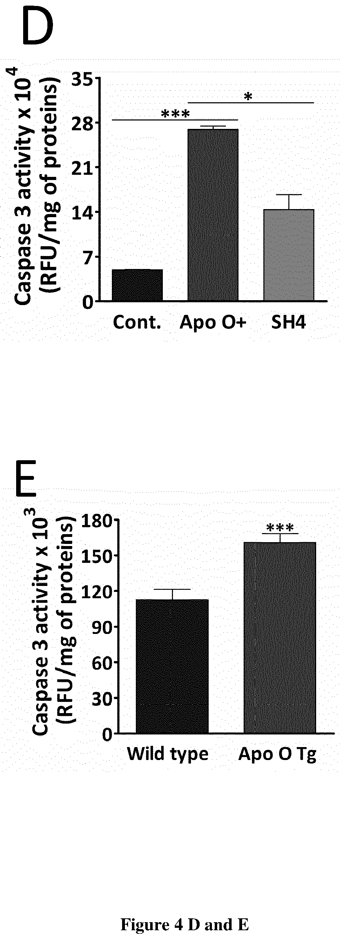

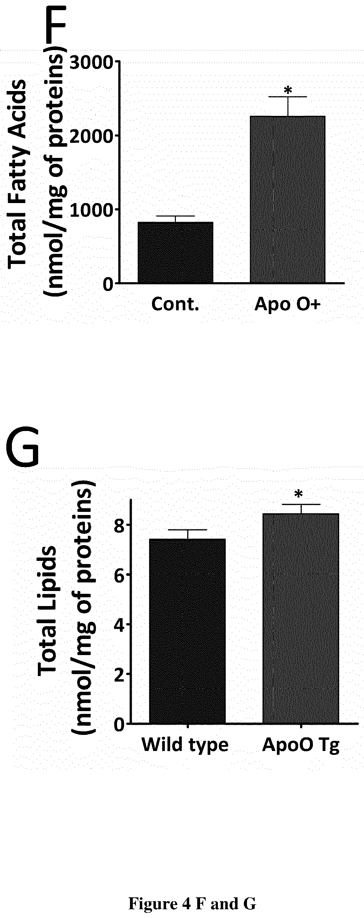

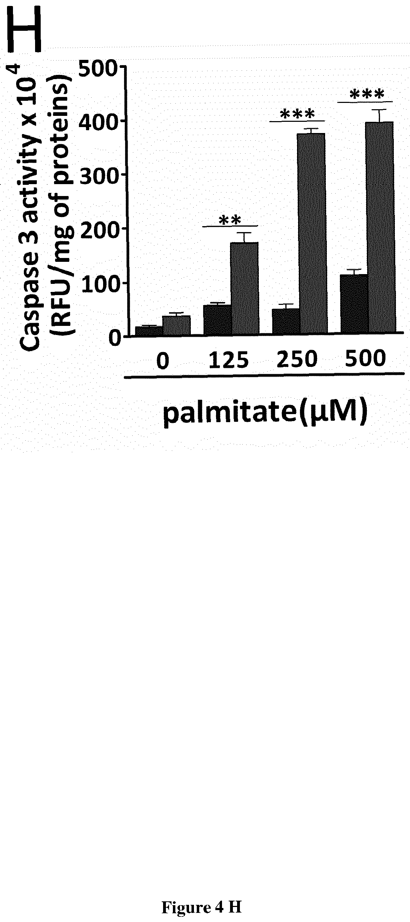

**Figure 4****: ApoO overexpression increases lipid accumulation and apoptosis**
   (A) Bax mRNA expression levels in control, Apo O+ and SH4 cardiac myoblasts and (B) their correlation to ApoO mRNA levels in ApoO Tg mouse or (C) in human hearts. (D) Caspase 3 activity in Apo O+ and SH4 cardiac myoblasts and (E) in wild type and ApoO transgenic mice. (F) Fatty acid levels in control and ApoO+ cardiac myoblasts. Cont.: control of stable transfectants generated by integration of pTT vector. Apo O+: stable integration of pTT-Apo O expression vector in H9c2 cardiac myoblasts manque SH4. (G) Comparison of total neutral lipid levels in wild type and ApoO Tg mice hearts. (H) Effect of exogeneous palmitate concentration on caspase 3 activity in control and Apo O+ cardiac myoblasts.

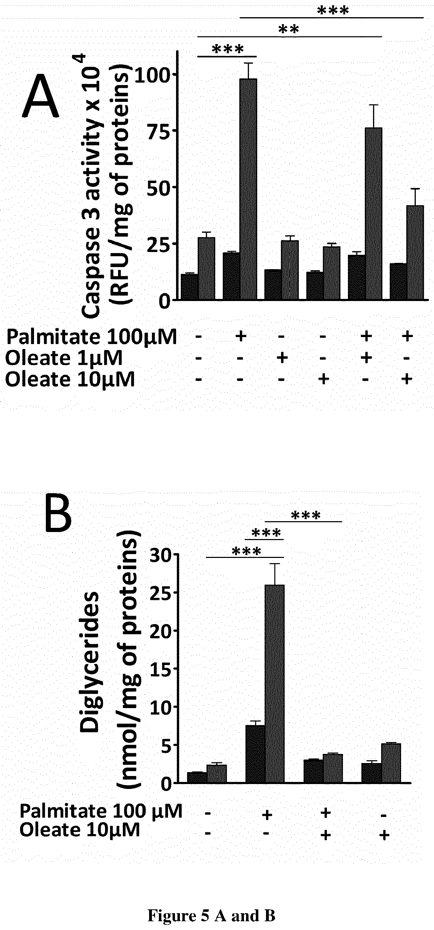

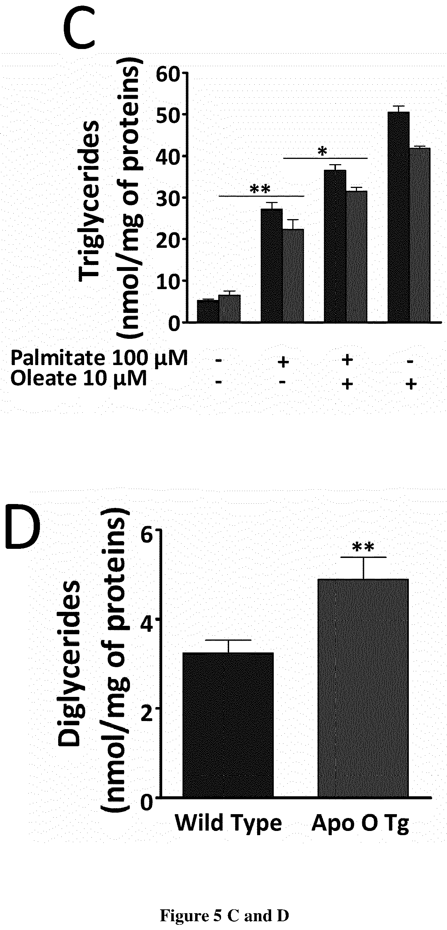

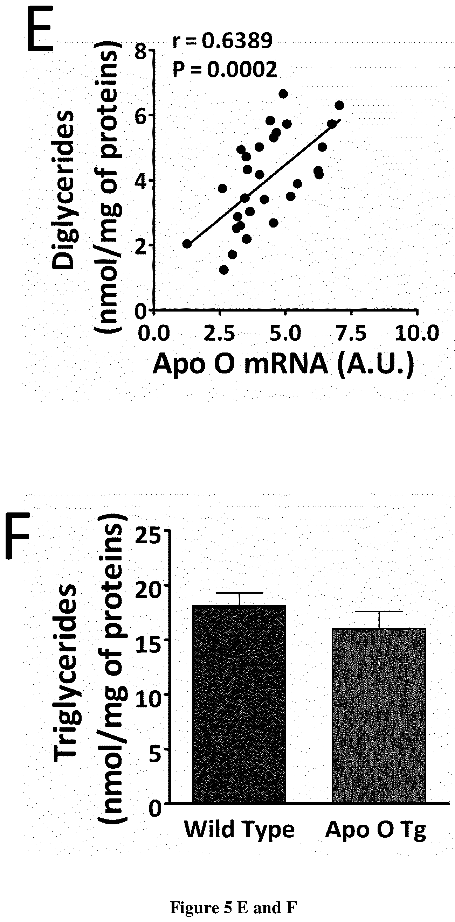

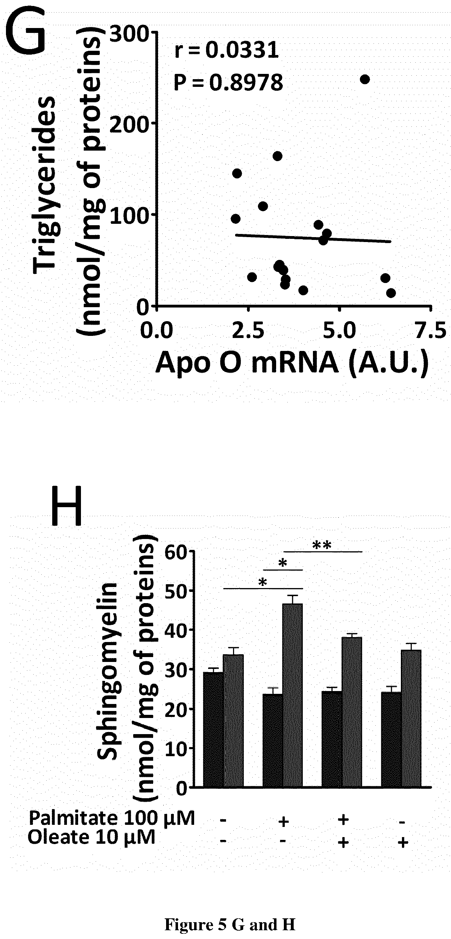

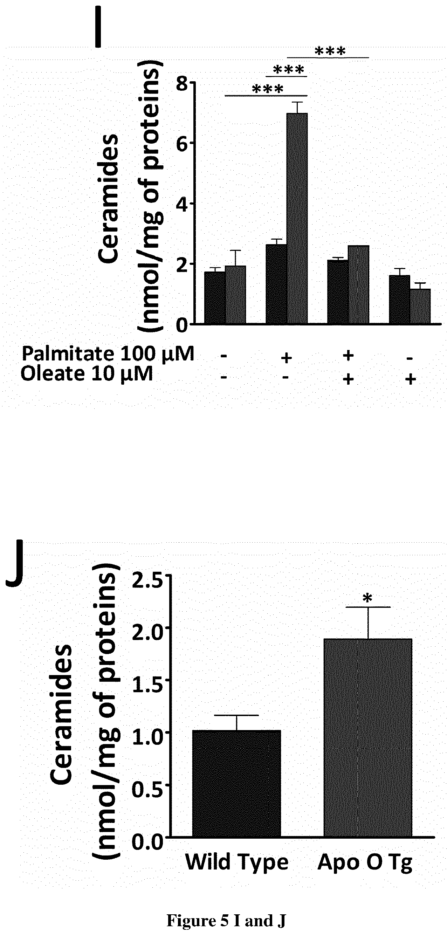

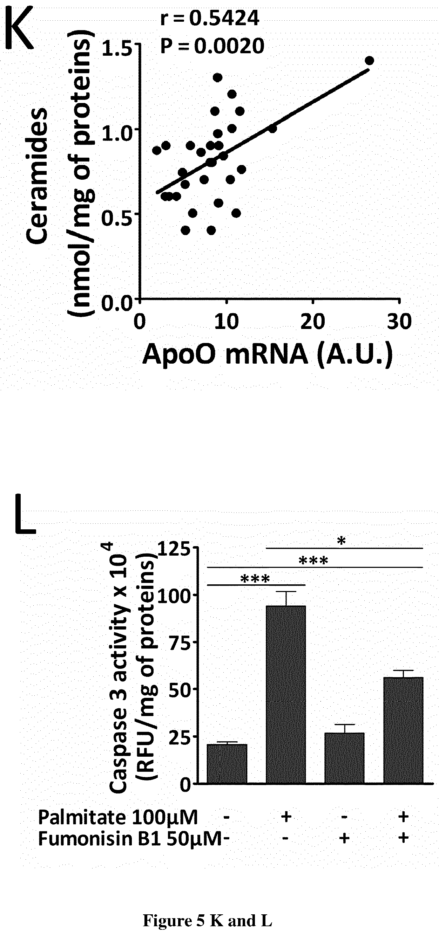

**Figure 5****: ApoO expression raises intracellular diglycerides but not triglycerides levels.**
   (A) Rescue of palmitate-induced cell death by oleate addition in control and Apo O+ cardiac myoblasts. (B) Effect of the rescue of palmitate-induced cell death by oleate on diglyceride levels. (C) Effect of the rescue of palmitate-induced cell death by oleate on triglyceride levels. (D) Diglycerides levels in heart of non-transgenic and ApoO Tg mice. (E) Correlation between intracardiac diglyceride levels and ApoO mRNA levels in the human heart. * p<0.05 ; **p<0.01, ***p<0.001. (F) Triglycerides levels in the heart of non-transgenic and ApoO Tg mice. (G) Lack of correlation between intracardiac triglyceride levels and ApoO mRNA levels in the human heart. Blue bars: control H9c2 cardiac myoblasts; red bars: ApoO+ H9c2 cardiac myoblasts. (H) Sphingomyelin levels in cells incubated with palmitate and/or oleate. (I) Ceramides levels in H9c2 cells incubated with palmitateand/or oleate. (J) Ceramides levels in ApoO transgenic and wild type mouse heart. (K) Positive correlation of ceramides levels with ApoO expression in human heart. (L) Caspase 3 activity in H9c2 cells incubated with palmitate, without or with the addition of fumonisin B 1.

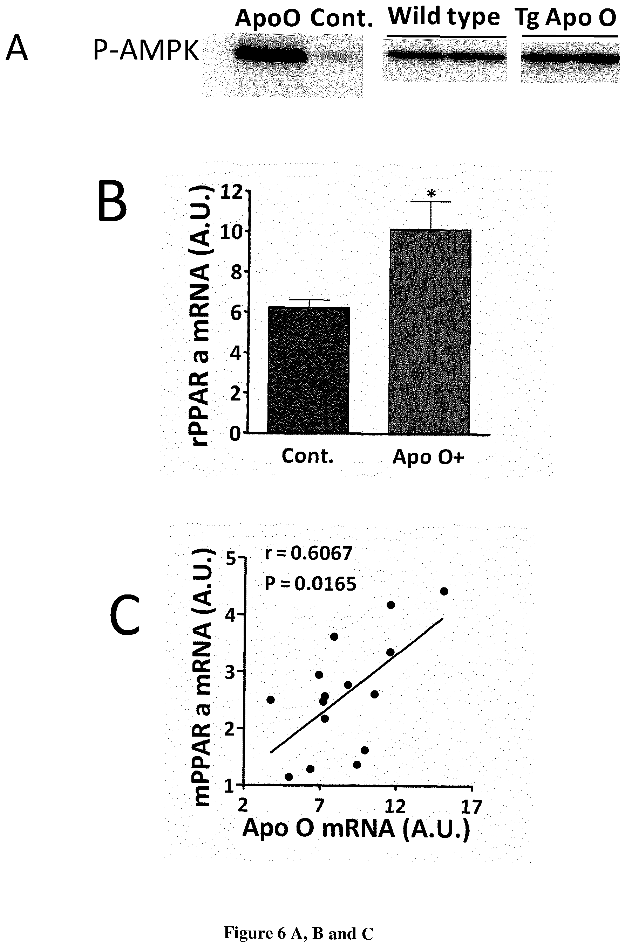

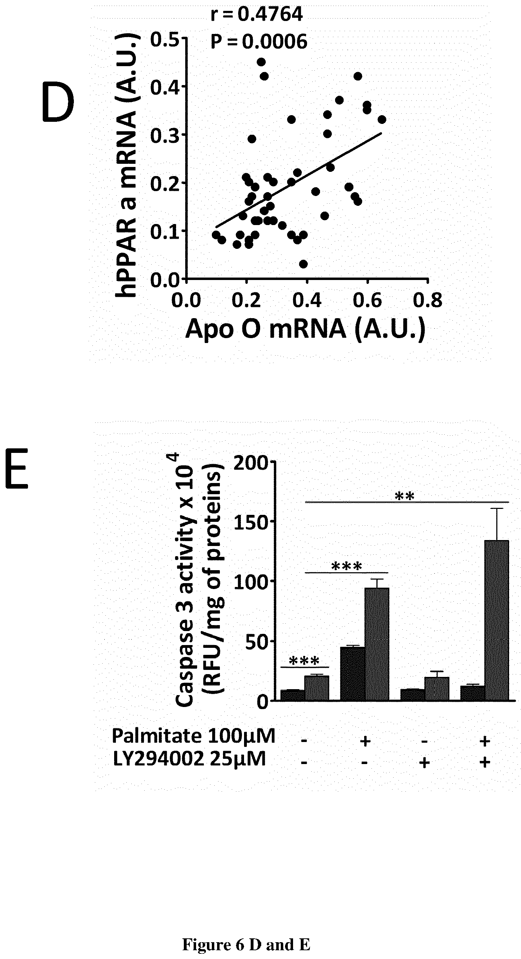

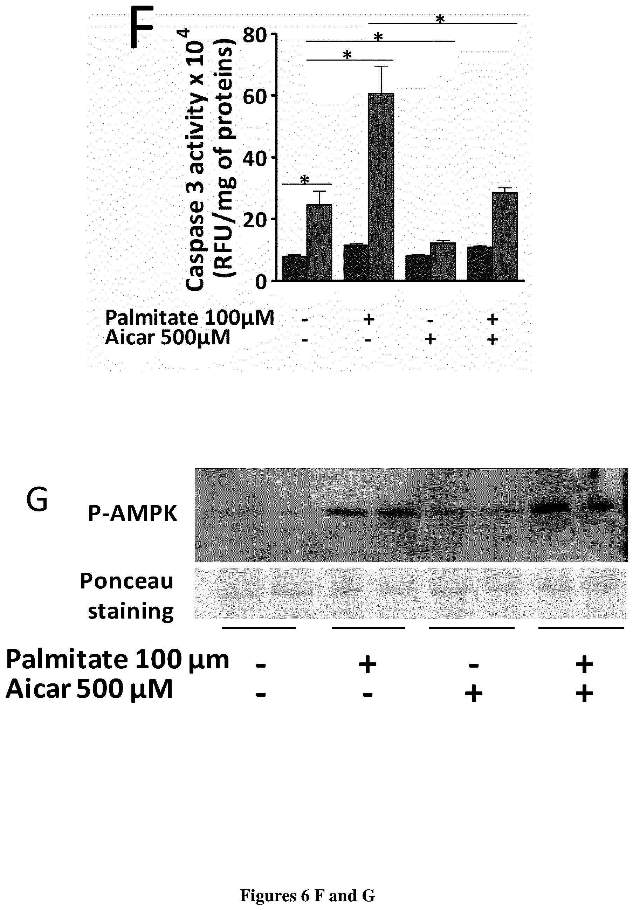

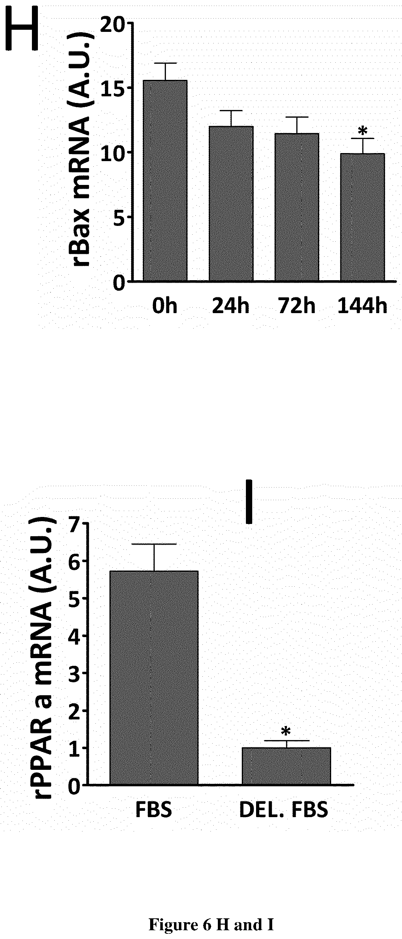

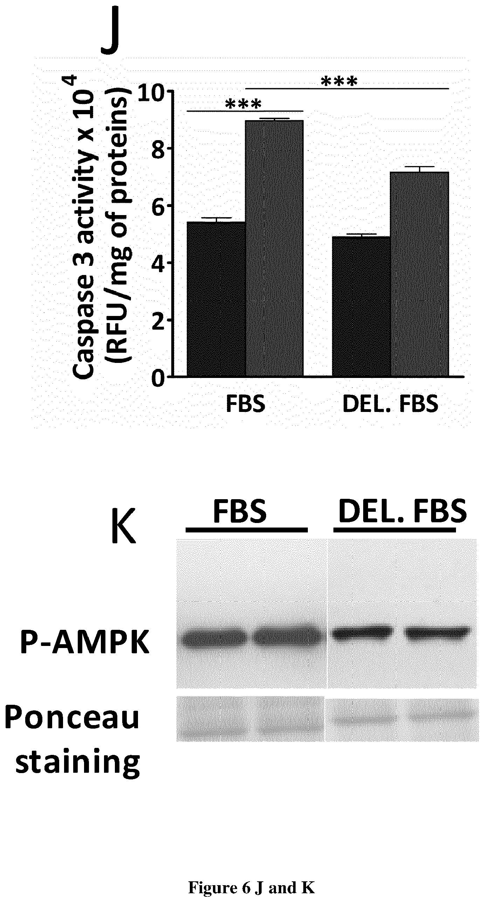

**Figure 6****: ApoO overexpression induces AMPK phosphorylation in the presence of lipids.**
   (A) Western blot analysis of AMPK phosphorylation. Left panel: control and Apo O+ cardiac myoblasts. Right panel: wild type and ApoO Tg mouse hearts. Two representative samples loading are shown (B) PPARα mRNA expression levels in control and ApoO+ cardiac myoblasts and (C) PPARα level correlation to ApoO mRNA levels in ApoO Tg mouse and (D) human hearts. (E) Caspase 3 activity in cells incubated with palmitate with or without LY294002. (F) Caspase 3 activity in cells incubated with palmitate with or without aminoimidazole carboxamide ribonucleotide (AICAR). (G) Western blot analysis of AMPK phosphorylation (P-AMPK) in ApoO+ cardiac myoblasts incubated with palmitate with or without AICAR. Consequences of fetal calf serum delipidation on (H) Bax and (I) PPARalpha expression; caspase 3 activity (J) and (K) AMPK phosphorylation after 12 hours of incubation in media supplemented with lipid-free fetal bovine serum (DEL FBS). Ponceau staining is provided to check for protein loading of the gel [Romero-Calvo 2010]. Time course of Bax expression in Apo O+ cells incubated in medium supplemented with lipid-free fetal bovine serum (center). Effect of lipid-free serum on PPARα gene expression (right).

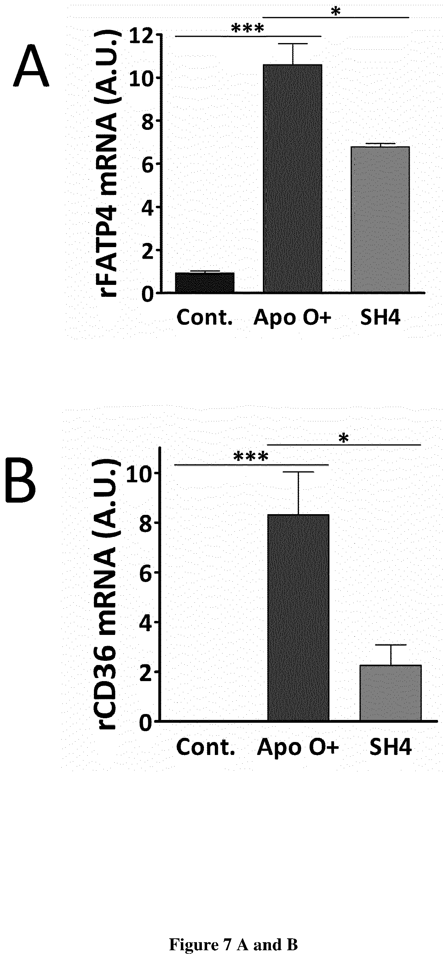

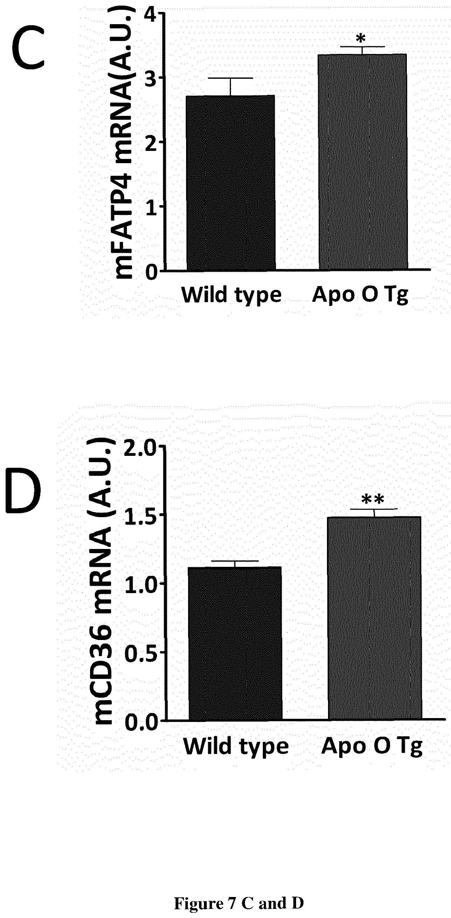

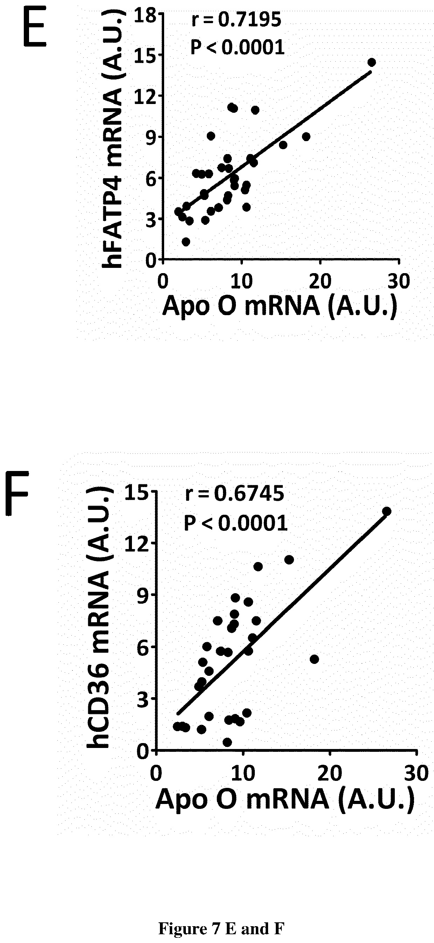

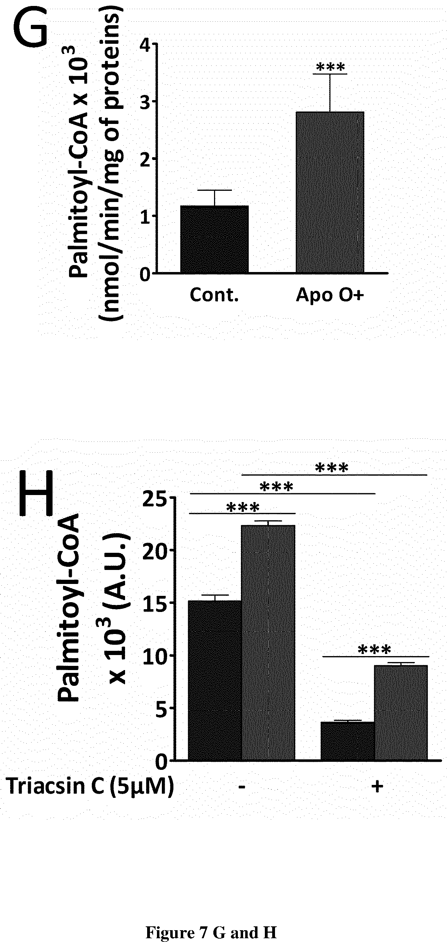

**Figure 7****: ApoO overexpression induces fatty acid transporters expression and acy-coA synthase activity.**
   FATP4 (A) and CD36 (B) mRNA level in control (cont.), ApoO+ and SH4 H9c2 cardiac myoblasts. FATP4 (C) and CD36 (D) mRNA level in wild type and ApoO transgénic mices (ApoO Tg). Positive correlation of ApoO mRNA level with hFATP4 (E) and hCD36 (F) mRNA level in human heart samples. (G) Palmitoyl-CoA activity in H9c2 control and ApoO+ H9c2 cardiac myoblasts. (H) Palmitoyl-CoA activity in H9c2 control and ApoO+ H9c2 cardiac myoblasts, incubated with (+) or without (-) Triacsin C.

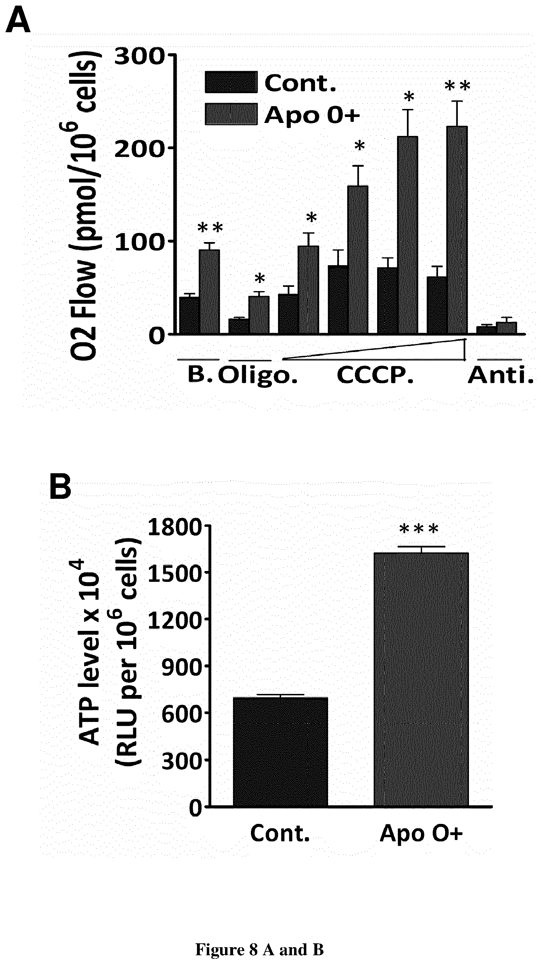

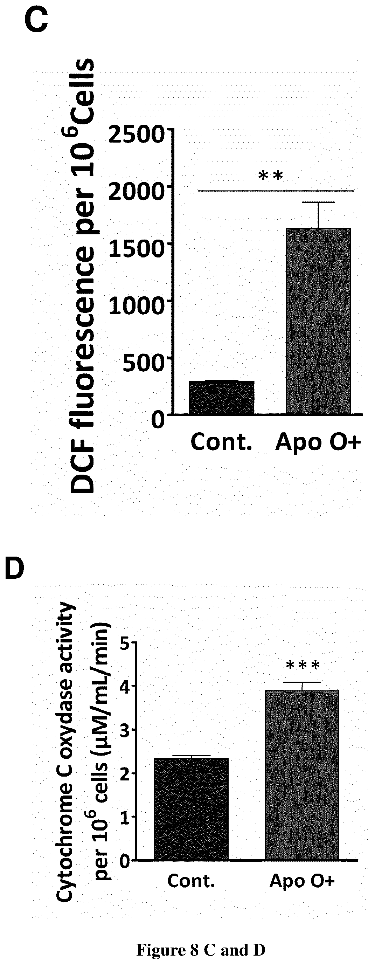

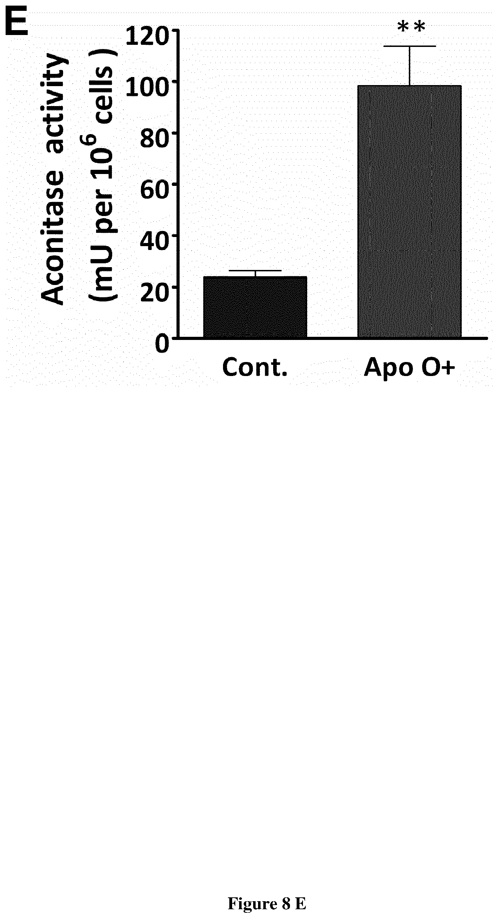

**Figure 8****: ApoO overexpression increases cell respiration, ATP and reactive oxygen species levels.**
   (A) Oxygen flow measurement in control (cont.) and ApoO+ H9c2 cardiac myoblasts. B: basal; Oligo: oligomycine two doses are shown ; CCCP: Carbonyl cyanide m-chlorophenyl hydrazone, 4 cumulatives doses are shown (4 to 16 µM) ; Anti: antimycin. (B) ATP level in control (cont.) and ApoO+ H9c2 cardiac myoblasts. (C) Reactive oxygen species measurement with 2' ,7' dichlorodihydrofluorescein diacetate (DCF) probe. (D) Cytochrome C oxydase activity in control (cont.) and ApoO+ H9c2 cardiac myoblasts. (E) Aconitase activity in control (cont.) and ApoO+ H9c2 cardiac myoblasts.

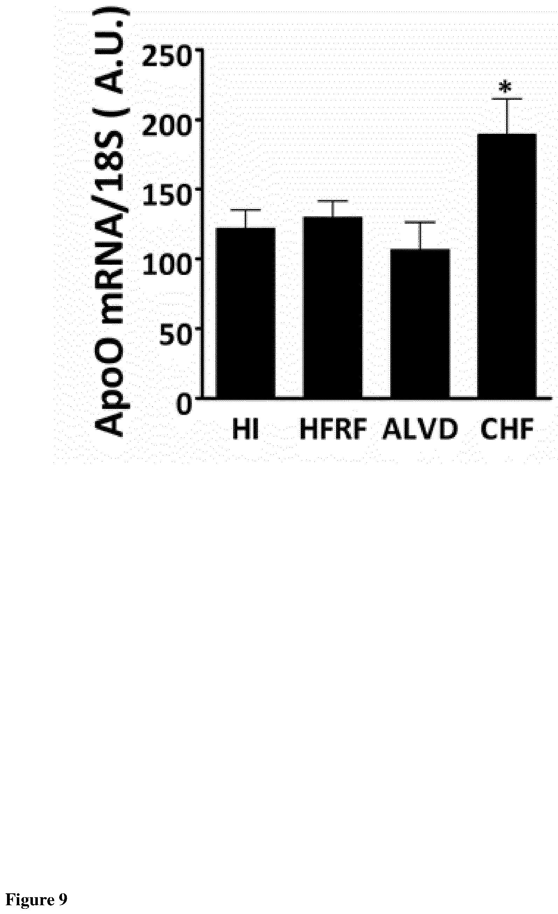

**Figure 9****: RT-Real-time PCR monitoring of ApoO mRNA level in white blood cells:**
   HI: healthy individuals (n=9); HFRF: heart failure risk factors patients (n= 13); ALVD: Asymptomatic Left Ventricular Dysfunction individuals (n=9); CHF: chronic heart failure patients (n=12). *p<0.05 : Students' t test CHF vs other groups. SEM are indicated.

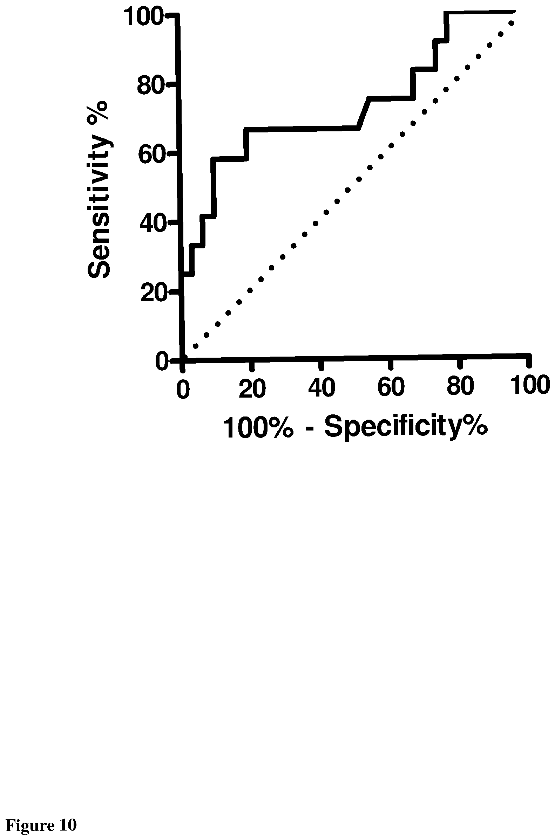

**Figure 10****: Evaluation of apoO as biomarker for Chronic Heart failure diagnosis by ROC analysis:**
   Receiver operating characteristics curve for ApoO expression level in white blood cells to test for patients with Chronic Heart Failure. Area under the curve is 0.73 with a significance level of P= 0.01 (n=43).

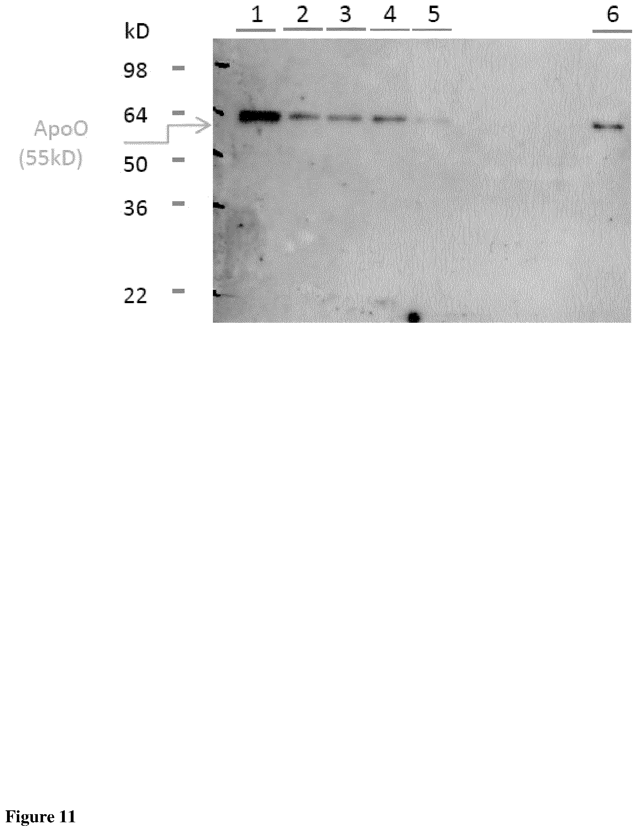

**Figure 11****: Western Blot of urine samples**
   90 µg of total desalted urine proteins were loaded onto a 11 % SDS-PAGE and detected using rabbit polyclonal antibodies (Lamant et al. 2006). ApoO (55kDa) is detected by Western Blot in 6 different urine samples that were tested with two preparation protocol (direct loading or after sample desalting which allowed for a better detection).

### EXAMPLES

### Material & Methods of examples

### Animals:

Studies followed the INSERM IFR150 Animal Facility guidelines. Moreover, the use of animals was approved by the animal care and use committees of INSERM I2MC UMR 1048. The generation of transgenic mouse models used here has been reported previously. All animal procedures were performed according to the guidelines of the French Ministry of Agriculture. Animals were housed at the Toulouse IFR31 animal facility in a room lit 12 h per day (6 AM-6 PM) at an ambient temperature of 22 +/- 1°C.

### Construction of ApoO expression vectors

To overexpress the human ApoO, the ApoO coding sequence was amplified using primers and cloned into the BamH1 site of pTT expression vector (Durocher, Perret et al. 2002). The cloned cDNAs in the resulting pTT-ApoO expression vector were verified by DNA sequencing using ABI PRISM®BigDye™ Terminator version 3.1 Ready reaction cycle sequencing kit (Applied Biosystems) and loaded on an ABI electrophoresis DNA sequencing instrument (Applied Biosystems). pSNAP-ApoO was generated by PCR amplification of pTT-ApoO using primers and cloned in the EcoRV site of pSNAP-tag® (Ozyme, Saint-Quentin-en-Yvelines, France).

### Cell culture and tranfection of H9C2 cardiac myoblasts

H9c2 were obtained from the European Collection of Cell Cultures (ECACC). H9c2 cells were cultured in Dulbecco's modified Eagle medium (Invitrogen) adjusted to contain 1.5 g/liter sodium bicarbonate and supplemented with antibiotic-antimycotic solution (Invitrogen) and 10% fetal bovine serum (FBS, Biowest). Cells were plated on 10-cm diameter tissue culture dish and grown in a 5% CO2 incubator at 37°C with saturating humidity with medium changes every 2 days. H9c2 cardiac myoblasts were stably transfected by electroporation and pools of tranfectant were selected as previously published (Smih, Rouet et al. 2002). FBS delipidation was performed as described (Sprong, Suchanek et al. 2006).

### Knock-down of ApoO overexpression

shRNA used to knock-down ApoO gene expression and controls were from MISSION shRNA set, i.e. Sh2=TRCN72707; Sh4= TRCN72704; Sh5=TRCN72705 (Sigma Aldrich). They are and used as recommended by generating pools of stable transfectants. Empty vector (no shRNA insert) control was also transfected and had no significant effect (not shown).

### Functional genomics

Total RNA were purified using TRIzol as recommended (Life Technologies SAS, Villebon sur Yvette) and further purified with RNeasy kit (Qiagen, Courtaboeuf) in a Qiacube (Qiagen, Courtaboeuf) automated protocol. Total RNA integrity was checked by Experion capillary electrophoresis (Bio-Rad, Marnes La Coquette). Samples with RNA Quality Indicator ≥8.5/10 were selected for analyses. Total RNAs were precisely quantified using RiboGreen and a Victor™ X5 2030 multilabel reader (Perkin Elmer, Courtaboeuf). Total RNA were used for fluorescent labelling using ChipShot™ Direct Labeling kit (Promega, Charbonnières-les-Bains). The labeled RNA were hybridized to pangenomic Operon (Qiagen design V3) rat glass microarrays displaying about 25,000 oligonucleotides probes. After standard hybridization, glass arrays were washed on a Ventana Discovery hybridization and wash system (Ventana Medical Systems SA, Illkirch) and scanned using a GenPix 4000 scanner (Molecular Devices France, St. Grégoire). Scanned images were processed by X-dot reader software (COSE, Paris) with operator's validation of the spots detection. Microarrays data were analyzed using both Toppgene (Chen, Bouman et al. 2009) and Ingenuity pathway analysis software (Ingenuity systems, Redwood City, CA, USA).

### Microarrays data mining

Microarray expression data from available human heart analysis, all generated using the Affymetrix U74A platform, were downloaded from the GEO database (Barrett, Troup et al. 2009). Arrays were intensity normalized and merged into one 90 arrays data set available. ApoO expression levels were plotted for the 90 human hearts tested and used to define the distribution of ApoO gene expression. A hierarchical clustering procedure (average group linkage, Pearson correlation, threshold r = 0.8) was applied to identify groups of co-expressed genes.

### Palmitate preparation and Caspase 3 activity monitoring

Palmitate preparation and caspase 3 activity measurement were performed as previously described (Hickson-Bick, Buja et al. 2000; Hirota, Otabe et al. 2006).

### Echocardiographic analysis

After shaving of the chest, echocardiograms were performed by using the Vivid 7 pro 7 echocardiographic system (GE Medical System), equipped with a i13 L 14-MHz linear-array transducer. Images were obtained from rats lightly anesthetized by 1-2% isoflurane (AErrane, Baxter) lying on their back side with transducer placed on the left hemithorax. Two-dimensional parasternal long- and short-axis images of the left ventricle were obtained, and two-dimensional targeted M-mode tracings were recorded at a sweep speed of 200 mm/s. All measurements were performed according to the recommendations of the American Society for Echocardiography leading-edge method from three consecutive cardiac cycles (n) with the roundness of the left ventricular cavity (2D-image) as a criterion that the image was on axis, great effort was taken to achieve a good image quality to visualize the endocardial and epicardial borders of the heart by gently moving and angulating the transducer. Measurements and calculations used are as follows: percent LV fractional shortening (FS), a measure of LV systolic function, was calculated as follows: FS=(EDD-ESD)/EDD×100, where EDD and ESD are end-diastolic and end-systolic diameters, respectively.

### Electrocardiogram

Surface electrocardiograms (ECGs) were recorded using an ADI system (ADIinstruments LTD). Q-T interval was corrected for 1 /2 heart rate using the formula: Q-Tc5Q-T/(R-R/ 100) established for mice with Q-T and R-R measured in milliseconds.

### Generation of human Apo O transgenic mice

Studies on transgenic mice were carried out in agreement with French laws and INSERM guidelines on animal care. The α-myosin heavy chain (αMHC)-ApoO transgene was constructed from a 5.5 kb BamHI-SalI fragment containing the murine αMHC promoter (Gulick, Subramaniam et al. 1991) and a SalI-Hind III cDNA fragment containing the human ApoO coding sequence (Lamant, Smih et al. 2006). The αMHC-ApoO transgene was linearized with NotI, purified by electroelution, concentrated on an elutip-d column (Schleicher and Schuell) and used for nuclear injection in fertilized eggs of B6D2/F1 hybrid females. The microinjected oocytes were then reimplanted in B6CBA/F1 hybrid pseudopregnant foster mothers. Three independent transgenic mice lines were generated and crossed with C57B616/J mouse strain. Genomic DNA was extracted using DNAeasy blood and tissue kit (Qiagen) in a QIAcube apparatus. Offspring were followed by PCR using the primers and Dynazyme II (New England Biolabs) enzyme. PCR were performed at least three times per mouse and PCR products were analyzed on acrylamide gels.

### Human heart tissue samples

After ethical committee approval, all patients included in the study gave their written consent for sample collection and molecular analysis prior to their inclusion. Patients were carefully selected by the physicians from the Department of Cardiology, Toulouse University Hospital, prior to cardiac surgery for coronary by-pass resulting from coronary disease. Right appendage were collected and immediately frozen in liquid nitrogen and kept at -80°C until analysis.

### RNA extractions and quality controls

Total RNA isolated from tissues samples; quality check and concentration control were performed as previously described (Philip-Couderc, Pathak et al. 2004). Total RNA were isolated from cultured H9c2 cardiac myoblasts using a RNeasy columns and QIAcube (Qiagen) automated apparatus according to the manufacturer's protocol.

### Real-time PCR analysis of gene expression

Oligos were designed with PerlPrimer (Marshall 2004) software and synthesized by Eurogentec Company. Real-time PCR was performed as described (Ruiz, Ordonez et al. 2003) in a MyiQ™ realtime PCR apparatus (Bio-Rad) using SurePrime kit reagents (Qbiogene). Real-time PCR was statistically analyzed with SigmaStat 3 software.

### Measurement of the mitochondria membrane potential

The mitochondria membrane potential was determined using flow cytometry using a Calibur FACS (BD biosciences) and the APO LOGIX JC-1 Mitochondrial Membrane Potential Detection Kit (Bachem).

### Confocal microscopy

Florescence detection of Bodipy-Palmitate was performed onto Falcon culture slides (BD Biosciences) (Fang, Palanivel et al. 2005). Subconfluent cells were washed twice in phosphate-buffered saline (PBS) and fixed in PBS containing 4% formaldehyde for 15 min at room temperature followed by 5 min at -20 °C. Cells were then washed x times in PBS and covered with a few drops of fluorescent mounting medium and coverslips before being analyzed on a Zeiss LSM 510 confocal microscope (Carl Zeiss).

### Monitoring of Apo O lipid level by NMR

Total lipids were analysed by 1H NMR spectroscopy as already published (Roncalli, Smih et al. 2007).

### Monitoring of Apo O lipid level by gas chromatography

Ceramides, diglycerides and triglycerides were quantified by gas chromatography as previously described (Vieu, Terce et al. 2002).

### Western blot analysis

Cardiac tissue was disrupted with mammalian MCL-1 cell lysis kit solution (Sigma Aldrich) in the presence of a mix of protease inhibitors, and procedures were performed according to the manufacturer's protocol. Sixty micrograms of protein was loaded on a 10% polyacrylamide-SDS gel that was blotted on a 0.45-µm nitrocellulose membrane BA85 (Schleicher and Schuell). MultiMark Multi-Colored standard (Invitrogen, Les Ulis, France) was used to determine size of the proteins. Nitrocellulose membranes were blocked for 2 h in TBS (7 mM Tris, pH 7.5; 150 mM NaCl) with 0.1% Tween 20 and 3% nonfat dry milk. Hybridization of the anti-TRIP-1 serum was performed in TBS-Tween 0.1% during 2 h. After three washes in TBS-Tween 0.1%, horseradish peroxidase conjugate (10-4 dilution) was incubated for 2 h with the membrane in TBS-Tween 0.1%, 3% nonfat dry milk. Blots were washed three times in Tween-TBS and one time in TBS; then hybridizations were revealed with SuperSignal West Pico chemiluminescent substrate according to the manufacturer's protocol (Perbio).

### Isolement of plasma membrane and mitochondria

Subfractionation of plasma membrane and mitochondria were performed using ultracentifugation as previously performed (Harmancey, Wilson et al.).

### Statistics

All results are depicted as means ± SEM. Multiple comparisons were analyzed using ANOVA followed, when appropriate, by the Dunnett post hoc test using Statview 4.5 software (Abacus Concepts). Single comparisons were performed using unpaired Student's t-test with a value of P 0.05 considered as significant.

### qPCR

Blood was collected in BD CPT tubes (BD biosciences) and processed as recommended by the manufacturer. RNA were extracted using RNeasy kit and a Qiacube automat (Qiagen) as recommanded. RT was performed using 500 ng of total RNA and Thermoscript (Invitrogen) as recommended. For qPCR Sybrgreen technology in a Bio-Rad MyIQ Cycler using the primers, the standard curve method was used for mRNA level measurement.

### Example 1: Apolipoprotein O is a mitochondrial protein which regulates energetic metabolism in cardiomyocytes

### Results

### ApoO is a mitochondrial protein associated with apoptosis regulation in H9c2 cardiac myoblasts

Human ApoO mRNA level was increased about 9 times by stable integration of the pTT-hApoO expression vector in H9c2 cardiac myoblasts to generate ApoO+ pools of stably integrated transfectants (Figure 1A). Subsequent, stable expression of hApoO shRNA in ApoO+ cells led to a reduced level of ApoO mRNA for SH4 and SH5 shRNA expression vectors but SH2 shRNA expression vector had no significant effect. As a consequence, we observed a reduction of the hApoO level by western blot analysis in SH4 clones (Figure 1B). We then investigated by western blot for the subcellular localization of ApoO and observed that ApoO is present both in membrane and in isolated mitochondria (Figure 1C) and was also detected in mitochondria by confocal laser microscopy using a fluorescent tagged ApoO. The latter showed a similar pattern of intracellular localization with Mitotracker, used as fluorescent markers of mitochondria (data not shown).

ApoO overexpression resulted in a profound alteration of the H9c2 transcriptome that was analyzed by microarrays analysis. We used hierarchical clustering representation to provide a global picture and to depict genes co-regulations that could be visualized in a group of clusters (data not shown). We further analysed the list of the upregulated genes using Toppgene software that proposed for biological process "death", "cell death" and "apoptosis" as major biological process significantly associated to ApoO overexpression (data not shown).

### ApoO expression level is correlated with expression of mitochondrial dysfunction related genes in human heart

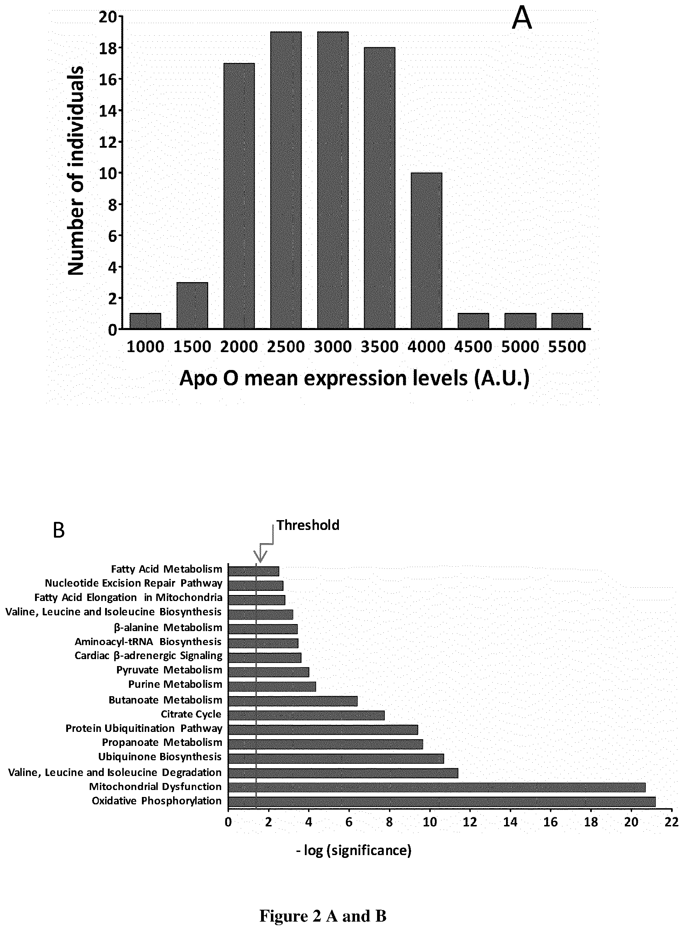

We analyzed data set from 90 microarrays performed with human heart samples and available in GEO database. We observed that ApoO mRNA level had an amplitude of about 5 fold depending on the patients tested (Figure 2A). Then, we noticed significant correlations of ApoO mRNA level with a number of genes that were sorted into pathways by Ingenuity pathway software. This analysis provided oxidative phosphorylation and mitochondrial dysfunction as major significant pathways associated to ApoO expression (Figure 2B). Moreover, we observed clearly a strong positive correlation of ApoO expression with oxidative phosphorylation and mitochondrial dysfunction genes (data not shown). Furthermore, comparison of the 15 higher ApoO expression level and the 15 lower showed that numerous genes encoding for mitochondrial proteins were significantly induced with ApoO rise in expression (data not shown).

### ApoO overexpression increases cell respiration, ATP synthesis and reactive oxygen species (ROS) production

Measurement of oxygen flow revealed a significantly increased oxygen consumption in ApoO+ H9c2 cardiac myoblasts which was further increased by the use of CCCP a strong uncoupler (Figure 8A). Moreover, ApoO+ displayed an increased ATP level (Figure 8B) and increased ROS production (Figure 8C). These phenomena were concomitant with increased cytochrome C oxydase (Figure 8D) and aconitase activity (Figure 8E).

### Example 2: Apolipoprotein O overexpression leads to cardiolipotoxicity and functional alterations

### Results

### ApoO overexpression leads to mitochondrial alterations, ventricular dilatation and ECG abnormalities

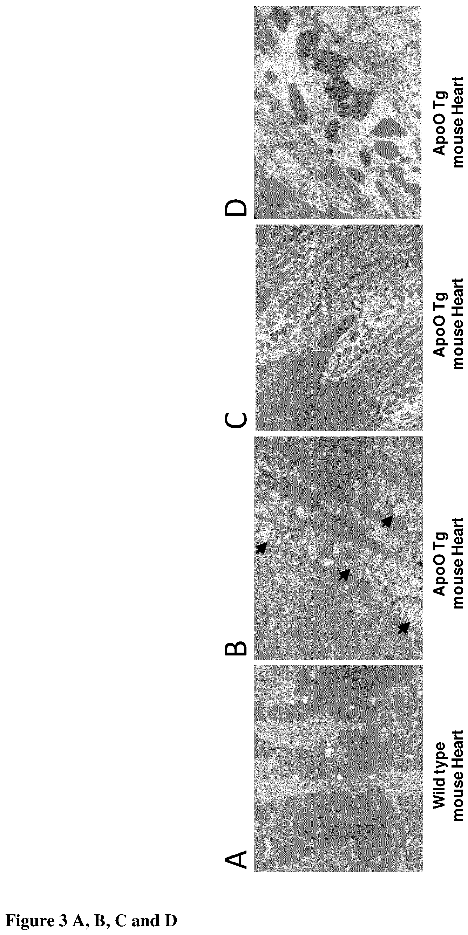

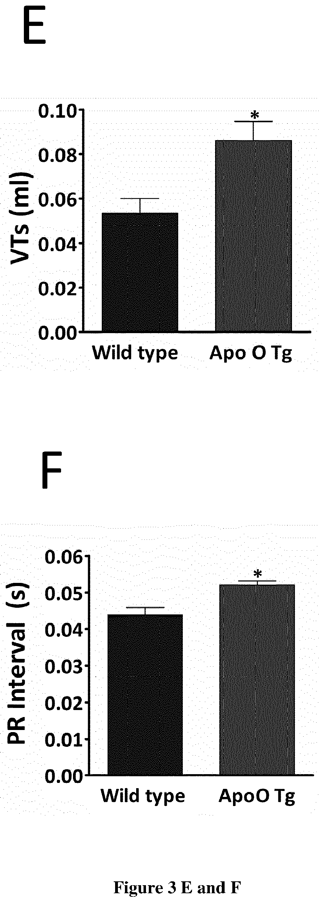

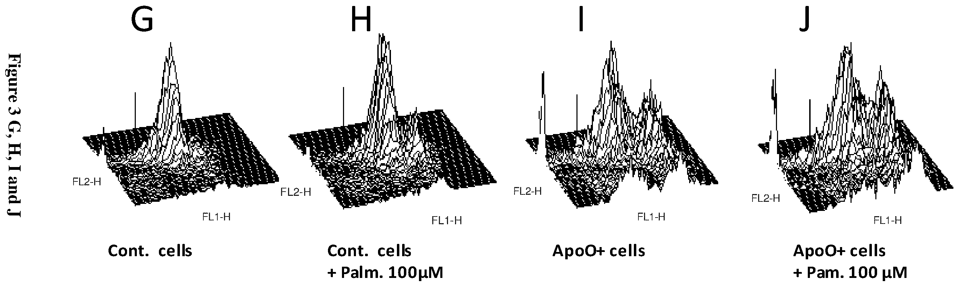

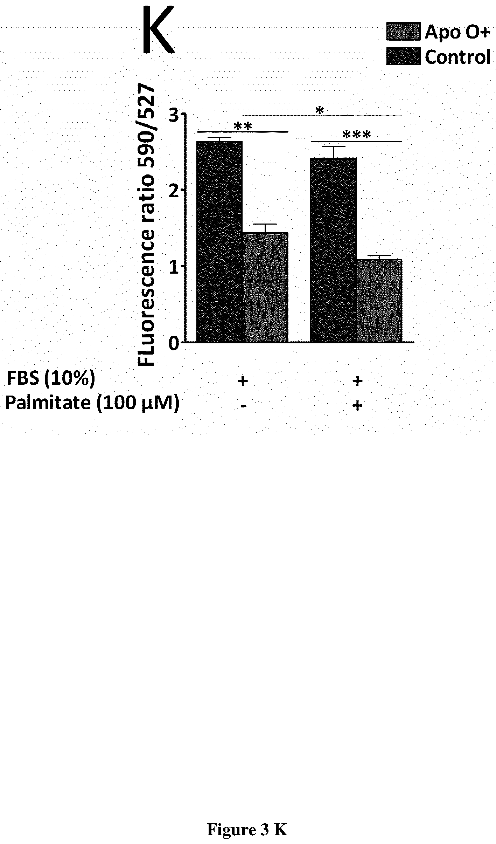

Transmission electron microscopy analysis of ApoO transgenic mice myocardium sections showed numerous altered mitochondria and tissular disorganization (Figure 3B, C, D) when compared to the non transgenic litter mate myocardium sections (Figure 3A). Echocardiographic analysis showed an increase in the left ventricle volume (Figure 3E) and electrocardiographic analysis revealed an increased PR interval (Figure 3F). Analysis by transmission electron microscopy of H9c2 palmitate treated cardiac myoblasts had no significant effect (not shown), whereas ApoO+ cells clearly showed mitochondrial alteration (data not shown). These mitochondrial damage resulted in the generation of multilamellar bodies (data not shown), indicative of concomitant autophagic stage. These autophagic mechanisms were further confirmed by transfecting a fluorescent GFP-LC3 protein (data not shown). Finally, we observed a drop in the mitochondrial transmembrane potential in ApoO+ H9c2 cardiac myoblasts (Figure 3I) that was further magnified by palmitate treatment (Figure 3J and K).

### ApoO expression level is positively correlated to apoptosis gene expression and lipid content level in cardiac myoblasts and hearts

ApoO overexpression led to a strong increase in Bax expression which was subsequently reduced by ApoO knock-down mediated by transfection of the SH4 plasmid (Figure 4A). Moreover, ApoO expression was positively correlated to Bax both in hearts from transgenic mice lines (Figure 4B) and in human heart (Figure 4C). As a consequence of ApoO overexpression we noticed an increased caspase 3 activity both in H9c2 cardiac myoblasts (Figure 4D) and in transgenic mice hearts (Figure 4E). We further observed a strong increase in intracellular total fatty acids in ApoO+ cardiac myoblasts (Figure 4F) and in total neutral lipids in transgenic mice hearts (Figure 4G). Use of bodipy palmitate and cofocal microscopy showed a strong accumulation of fluorescent palmitate in ApoO+ cardiac myoblasts (not shown) when compared to control cells. Incubation of Apo O+ or control H9c2 cardiac myoblasts with increased amounts of palmitate resulted in a strong increase of caspase 3 activity in ApoO+ but had no significant effects in control cell excepted for high palmitate concentration (500 µM, Figure 4 H).

### ApoO overexpression leads to diglycerides and ceramides accumulation in rat cardiomyoblast, transgenic mice and human hearts

ApoO overexpression led to a strong rise in caspase 3 activity in ApoO+ cells which was reversed by oleate addition (Figure 5A). Measurement of intracellular lipids showed an accumulation of diglycerides that was reversed by addition of oleate (Figure 5B). Triglycerides content was not significantly affected by ApoO overexpression and was slightly lowered by oleate addition (Figure 5C). Transgenic mice overexpressing ApoO in the myocardium also displayed diglycerides accumulation (Figure 5D). Moreover, we observed a significant positive correlation between ApoO mRNA level and diglycerides accumulation in human heart (Figure 5E). As observed in cell culture, modulations of the ApoO expression level did not significantly modified the triglyceride level in ApoO transgenic mice nor in human heart (Figures 5F and G). Measurement of sphingomyelin and ceramides showed a significant rise in concentration for these molecules in Apo O+ cardiomyoblastes (Figure 5H and I), in ApoO transgenic mice (Figure 5J) and in human heart (Figure 5K). Inhibition of ceramide synthesis by Fumonisin B1 showed a lowered caspase 3 activity (Figure 5L).

### AMPK pathway is involved in ApoO mediated lipoapoptosis.

Western blot analysis showed an increased AMPK phosphorylation in H9c2 cardiac myoblasts overexpressing ApoO and in MHC-ApoO transgenic mice hearts (Figure 6A). We observed a rise in PPARalpha mRNA level in ApoO+ cells (Figure 6B), and a significant positive correlation of levels of PPARalpha mRNA with ApoO mRNA in the 3 transgenic mice lines (Figure 6C) and in human heart (Figure 6D). Moreover, addition of LY294002 a potent and specific cell-permeable inhibitor of phosphatidylinositol 3-kinases (PI 3-kinases) had no significant effect on caspase 3 activity (Figure 6E) whereas addition of AICAR (5-Aminoimidazole-4-carboxyamide ribonucleoside), an adenosine analog which stimulates AMPK activity significantly reduced caspase 3 activity (Figure 6F). Western blot analysis phosphorylation revealed that AICAR increased AMPK phosphorylation (Figure 6G), even when palmitate was simultaneously added. Moreover, analysis of gene expression showed that both Bax, a well-known apoptosis related gene and PPARα mRNA level were lowered by serum delipidation (Figure 6I and J, respectively). Caspase 3 activity was also reduced (Figure 6J). Western blot analysis of ApoO+ cells incubated in lipid free serum showed a reduced level of phosphorylated AMPK (Figure 6K).

### ApoO mediated AMPK pathway activation leads to fatty acids transporters mRNA level induction and increased palmitoyl-coA activity

FATP4 and CD36 mRNA levels were significantly increased by ApoO overexpression both in H9c2 cardiac myoblasts (Figure 7A and B) and in transgenic mice hearts (Figure 7C and D). These up-regulation were reduced by the ApoO knock-down in SH4 cells (Figure 7A and B). Moreover, gene expression analysis in human heart samples showed a significant positive correlation of ApoO expression with FATP4 and CD36 (Figure 7E and F). Measurement of palmitoyl-coA synthase activity, an enzymatic activity featured by FATP4 and CD36 fatty acid transporters (Jia, Pei et al. 2007), revealed a strong increase of palmitoyl-coA activity which was inhibited by triacsin C, a potent inhibitor of long fatty acyl CoA synthetase (Figure 7G and H).

### Example 3: Apolipoprotein O is a chronic heart failure biomarker

As shown in figures 9 and 10, ApoO mRNA level is significantly induced in white blood cells from patients with chronic heart failure when compared to white blood cells expression levels in blood samples from healthy individuals, heart failure risk factor individual and asymptomatic left ventricular dysfunction individuals.

### Exemple 4: ApoO leads to intracellular accumulation of lipotoxic species in heart and liver

We have shown that ApoO leads to intracellular accumulation of lipotoxic species in cardiomyocyes as well as in the liver.

Triglyceride levels were not significantly modified whereas diglyceride and ceramide levels were increased by expression of ApoO in murine heart or liver. Similarly, in human right atrial appendage samples, endogenous ApoO mRNA levels correlated with diglyceride and ceramide levels, but not with triglyceride levels. Moreover, palmitate treatment of cardiac myoblasts expressing ApoO induced a dramatic intracellular accumulation of diglycerides and did not have a significant effect, though, on triglyceride levels when compared to control cells. Palmitate treatment also increased ceramides levels and caspase-3 activity that was partially attenuated by the ceramide synthase inhibitor, fumonisine B.

As previously mentioned, we showed that in cardiac myoblasts overexpressing ApoO, intracellular content of fatty acids was increased, and while escalating doses of palmitate moderately increase caspase-3 activity in normal cells, ApoO expression dramatically amplified this effect. In addition, ApoO overexpression led to both faster palmitate uptake rates and lipid accumulation within the cells. Fatty acid uptake may have exceeded mitochondrial fatty acid oxidative capacity, thus resulting in increased lipid storage, thereby exerting a lipotoxic effect. Accordingly, in all our ApoO expressing models, including the human heart, cells displayed an impressive accumulation of toxic lipids such as diglycerides and ceramides.

This was shown, as previously mentioned in cardiomyocytes. However, we also demonstrated an accumulation of lipid in the liver. Therefore, these results indicate that increased ApoO expression induces lipotoxic mechanisms.

### Exemple 5 : ApoO is detectable in urine sample

The inventors performed a Western Blot on 6 different urine samples, said samples being obtained in 6 subjects used to test for detection of ApoO in urine.

As shown in Figure 11, the inventors have evidenced the presence of ApoO in said samples.

Therefore, the inventors have shown that the method of the invention may easily be implemented on urine sample.

### REFERENCES:

Throughout this application, various references describe the state of the art to which this invention pertains.
Dickstein K, Cohen-Solal A, Filippatos G, McMurray JJ, Ponikowski P, Poole-Wilson PA, Strömberg A, van Veldhuisen DJ, Atar D, Hoes AW, Keren A, Mebazaa A, Nieminen M, Priori SG, Swedberg K; ESC Committee for Practice Guidelines (CPG). ESC guidelines for the diagnosis and treatment of acute and chronic heart failure 2008: the Task Force for the diagnosis and treatment of acute and chronic heart failure 2008 of the European Society of Cardiology. Developed in collaboration with the Heart Failure Association of the ESC (HFA) and endorsed by the European Society of Intensive Care Medicine (ESICM). Eur J Heart Fail. 2008 Oct;10(10):933-89. Epub 2008 Sep 16.
Barrett, T., D. B. Troup, et al. (2009). "NCBI GEO: archive for high-throughput functional genomic data." Nucleic Acids Res 37(Database issue): D885-890.
Chen, C., T. J. Bouman, et al. (2009). "A systematic approach to evaluate humoral and cellular immune responses to Coxiella burnetii immunoreactive antigens." Clin Microbiol Infect 15 Suppl 2: 156-157.
Durocher, Y., S. Perret, et al. (2002). "High-level and high-throughput recombinant protein production by transient transfection of suspension-growing human 293-EBNA1 cells." Nucleic Acids Res 30(2): E9.
Fang, X., R. Palanivel, et al. (2005). "Hyperglycemia- and hyperinsulinemia-induced alteration of adiponectin receptor expression and adiponectin effects in L6 myoblasts." J Mol Endocrinol 35(3): 465-476.
Gulick, J., A. Subramaniam, et al. (1991). "Isolation and characterization of the mouse cardiac myosin heavy chain genes." J Biol Chem 266(14): 9180-9185.
Harmancey, R., C. R. Wilson, et al. (2010). "Western diet changes cardiac acyl-CoA composition in obese rats: a potential role for hepatic lipogenesis." J Lipid Res 51(6): 1380-1393.
Hickson-Bick, D. L., L. M. Buja, et al. (2000). "Palmitate-mediated alterations in the fatty acid metabolism of rat neonatal cardiac myocytes." J Mol Cell Cardiol 32(3): 511-519.
Hirota, N., S. Otabe, et al. (2006). "Sequential activation of caspases and synergistic beta-cell cytotoxicity by palmitate and anti-Fas antibodies." Life Sci 79(13): 1312-1316.
Jia, Z., Z. Pei, et al. (2007). "The fatty acid transport protein (FATP) family: very long chain acyl-CoA synthetases or solute carriers?" J Mol Neurosci 33(1): 25-31.
Lamant, M., F. Smih, et al. (2006). "ApoO, a novel apolipoprotein, is an original glycoprotein up-regulated by diabetes in human heart." J Biol Chem 281(47): 36289-36302.
Marshall, O. J. (2004). "PerlPrimer: cross-platform, graphical primer design for standard, bisulphite and real-time PCR." Bioinformatics 20(15): 2471-2472.
Philip-Couderc, P., A. Pathak, et al. (2004). "Uncomplicated human obesity is associated with a specific cardiac transcriptome: involvement of the Wnt pathway." FASEB J 18(13): 1539-1540.
Roncalli, J., F. Smih, et al. (2007). "NMR and cDNA array analysis prior to heart failure reveals an increase of unsaturated lipids, a glutamine/glutamate ratio decrease and a specific transcriptome adaptation in obese rat heart." J Mol Cell Cardiol 42(3): 526-539.
Ruiz, V., R. M. Ordonez, et al. (2003). "Unbalanced collagenases/TIMP-1 expression and epithelial apoptosis in experimental lung fibrosis." Am J Physiol Lung Cell Mol Physiol 285(5): L1026-1036.
Smih, F., P. Rouet, et al. (2002). "Transcriptional regulation of adipocyte hormone-sensitive lipase by glucose." Diabetes 51(2): 293-300.
Sprong, H., M. Suchanek, et al. (2006). "Aberrant receptor-mediated endocytosis of Schistosoma mansoni glycoproteins on host lipoproteins." PLoS Med 3(8): e253.
Vieu, C., F. Terce, et al. (2002). "Coupled assay of sphingomyelin and ceramide molecular species by gas liquid chromatography." J Lipid Res 43(3): 510-522.

## Claims

1. A method for diagnosis chronic heart failure, acute heart failure or diabetic cardiomyopathy in a patient comprising :
➢ determining the expression level of Apolipoprotein O (Apo O) in a sample obtained from said patient; and
➢ comparing said expression level to a threshold value.

2. The method according to claim 1, wherein said threshold value is the mean expression level of ApoO of a population of healthy individuals, preferably 100 healthy individuals.

3. The method according to claim 1 or 2, wherein said sample is selected in the group consisting of blood, plasma, serum, lymph and urine sample.

4. The method according to any one of claims 1 to 3, wherein Apo O expression level is measured by quantifying the level of mRNA of Apo O gene in said sample.

5. The method according to claim 4, wherein Apo O expression level is measured by real-time quantitative or semi-quantitative RT-PCR.

6. The method according to any one of claims 1 to 3, wherein Apo O expression level is measured by quantifying the level of Apo O protein in the sample.

7. The method according to claim 6, wherein the quantification of the level of Apo O protein is performed by using a set of antibodies directed against Apo O.

8. The method according to claim 7, wherein the quantification of the level of Apo O protein is performed by immunohistochemistry.

9. An antisense oligonucleotide, siRNA, shRNA or ribozyme nucleic acid sequence which is an inhibitor of the Apo O gene expression for use in the treatment of chronic heart failure, acute heart failure or diabetic cardiomyopathy.

10. A compound for use according to claim 9 wherein said compound is a shRNA.

11. A pharmaceutical composition for use for the treatment of chronic heart failure, acute heart failure or diabetic cardiomyopathy, comprising a compound according to claim 9 or 10 and a pharmaceutically acceptable carrier.

## Patentansprüche

1. Ein Verfahren zur Diagnose chronischen Herzversagens, akuten Herzversagens und diabetischer Kardiomyopathie in einem Patienten umfassend:
➢ Bestimmen des Expressionsniveaus von Apolipoprotein O (Apo O) in einer Probe, die von dem Patienten erhalten wurde; und
➢ Abgleich dieses Expressionsniveaus mit einem Schwellenwert.

2. Verfahren nach Anspruch 1, wobei dieser Schwellenwert das mittlere Expressionsniveau von Apo O einer Population von gesunden Individuen, vorzugsweise 100 gesunden Individuen, ist.

3. Verfahren nach Anspruch 1 oder 2, wobei diese Probe ausgewählt ist aus der Gruppe, bestehend aus Blut, Plasma, Serum, Lymphe und einer Urinprobe.

4. Verfahren nach einem der Ansprüche 1 bis 3, wobei das Apo O-Expressionsniveau dadurch gemessen wird, dass das Niveau von mRNA des Apo O-Gens in dieser Probe quantifiziert wird.

5. Verfahren nach Anspruch 4, wobei das Apo O-Expressionsniveau gemessen wird durch eine quantitative oder semiquantitative Echtzeit-RT-PCR.

6. Verfahren nach einem der Ansprüche 1 bis 3, wobei das Apo O-Expressionsniveau dadurch gemessen wird, dass das Niveau des Apo O-Proteins in der Probe quantifiziert wird.

7. Verfahren nach Anspruch 6, wobei die Quantifizierung des Niveaus des Apo O-Proteins durch Verwendung eines Satzes von Antikörpern, die gegen Apo O gerichtet sind, ausgeführt wird.

8. Verfahren nach Anspruch 7, wobei die Quantifizierung des Niveaus des Apo O-Proteins durch Immunohistochemie ausgeführt wird.

9. Ein Antisense-Oligonukleotid, siRNA, shRNA oder eine Ribozym-Nukleinsäuresequenz, die ein Inhibitor der Apo O-Genexpression ist, zur Verwendung bei der Behandlung von chronischem Herzversagen, akutem Herzversagen oder diabetischer Kardiomyopathie.

10. Eine Verbindung zur Verwendung nach Anspruch 9, wobei diese Verbindung eine shRNA ist.

11. Eine pharmazeutische Zusammensetzung zur Verwendung bei der Behandlung von chronischem Herzversagen, akutem Herzversagen oder diabetischer Kardiomyopathie, umfassend eine Verbindung nach Anspruch 9 oder 10 und einen pharmazeutisch akzeptablen Träger.

## Revendications

1. Procédé de diagnostic d'une insuffisance cardiaque chronique, d'une insuffisance cardiaque aiguë ou d'une cardiomyopathie diabétique chez un patient comprenant :
- la détermination du niveau d'expression de l'Apolipoprotéine O (Apo O) dans un échantillon obtenu auprès dudit patient ; et
- la comparaison dudit niveau d'expression à une valeur seuil.

2. Procédé selon la revendication 1, dans lequel ladite valeur seuil est le niveau d'expression moyen d'Apo O d'une population d'individus sains, de préférence 100 individus sains.

3. Procédé selon la revendication 1 ou 2, dans lequel ledit échantillon est choisi dans le groupe constitué des échantillons de sang, de plasma, de sérum, de lymphe et d'urine.

4. Procédé selon l'une quelconque des revendications 1 à 3, dans lequel le niveau d'expression de l'Apo O est mesuré en quantifiant le niveau d'ARNm du gène Apo O dans ledit échantillon.

5. Procédé selon la revendication 4, dans lequel le niveau d'expression de l'Apo O est mesuré par RT-PCR quantitative en temps réel ou semi-quantitative.

6. Procédé selon l'une quelconque des revendications 1 à 3, dans lequel le niveau d'expression de l'Apo O est mesuré en quantifiant le niveau de protéine Apo O dans l'échantillon.

7. Procédé selon la revendication 6, dans lequel la quantification du niveau de protéine Apo O est réalisée en utilisant un ensemble d'anticorps dirigés contre Apo O.

8. Procédé selon la revendication 7, dans lequel la quantification du niveau de protéine Apo O est réalisée par immunohistochimie.

9. Séquence d'oligonucléotide antisens, de pARNi, d'ARNsh ou d'acide nucléique de ribozyme, qui est un inhibiteur de l'expression du gène de l'Apo O destiné à une utilisation dans le traitement de l'insuffisance cardiaque chronique, de l'insuffisance cardiaque aiguë ou d'une cardiomyopathie diabétique.

10. Composé pour une utilisation selon la revendication 9, dans lequel ledit composé est un ARNsh.

11. Composition pharmaceutique destinée à une utilisation pour le traitement de l'insuffisance cardiaque chronique, de l'insuffisance cardiaque aiguë ou d'une cardiomyopathie diabétique, comprenant un composé selon la revendication 9 ou 10 et un véhicule pharmaceutiquement acceptable.
